# EUROPEAN PATENT APPLICATION

(11) **EP 3 995 130 A1**
(43) Date of publication of application: **11.05.2022**
(21) Application number: 20206287.3
(22) Date of filing: 06.11.2020
(51) Int. Cl.: A61K 9/00, A61K 35/28, A61K 38/57, A61P 31/12

(54) **A COMPOSITION COMPRISING EXTRA-CELLULAR VESICLES FROM MESENCHYMAL STEM CELLS AND ALPHA-1 ANTITRYPSIN FOR THE TREATMENT OF VIRAL INFECTIONS**

(71) Applicant: JunctuCell Biomed Manufacturing GmbH, 85662 Hohenbrunn (DE)
(72) Inventor: Stangl, Manfred, 82054 Sauerlach (DE); Strassmair, Michael, 85662 Hohenbrunn (DE)
(74) Representative: Hertin und Partner Rechts- und Patentanwälte PartG mbB

(57) **Abstract**

The invention relates to a pharmaceutical composition comprising extra-cellular vesicles isolated from mesenchymal stem cells (MSCs) and Alpha-1 antitrypsin (AAT) protein, for use as a medicament in the treatment and/or prevention of a viral infection in a human subject. The invention further relates to the composition for topical and/or local administration to a mucosal surface of the respiratory tract and/or in the oral cavity of the subject. In another aspect the invention relates to a delivery system and corresponding formulations for dispensing the pharmaceutical composition.

SEQUENCE LISTING

<110>  JunctuCell Biomed Manufacturing GmbH

<120>  A COMPOSITION COMPRISING EXTRA-CELLULAR VESICLES FROM MESENCHYMAL
        STEM CELLS AND ALPHA-1 ANTITRYPSIN FOR THE TREATMENT OF VIRAL
       INFECTIONS

<130>  XII 2331/20EP

<160>  3

<170>  PatentIn version 3.5

<210>  1
<211>  1257
<212>  DNA
<213>  Homo sapiens

<400>  1
atgccgtctt ctgtctcgtg gggcatcctc ctgctggcag gcctgtgctg cctggtccct      60

gtctccctgg ctgaggatcc ccagggagat gctgcccaga agacagatac atcccaccat     120

gatcaggatc acccaacctt caacaagatc acccccaacc tggctgagtt cgccttcagc     180

ctataccgcc agctggcaca ccagtccaac agcaccaata tcttcttctc cccagtgagc     240

atcgctacag cctttgcaat gctctccctg gggaccaagg ctgacactca cgatgaaatc     300

ctggagggcc tgaatttcaa cctcacggag attccggagg ctcagatcca tgaaggcttc     360

caggaactcc tccgtaccct caaccagcca gacagccagc tccagctgac caccggcaat     420

ggcctgttcc tcagcgaggg cctgaagcta gtggataagt ttttggagga tgttaaaaag     480

ttgtaccact cagaagcctt cactgtcaac ttcggggaca ccgaagaggc caagaaacag     540

atcaacgatt acgtggagaa gggtactcaa gggaaaattg tggatttggt caaggagctt     600

gacagagaca cagtttttgc tctggtgaat tacatcttct ttaaaggcaa atgggagaga     660

ccctttgaag tcaaggacac cgaggaagag gacttccacg tggaccaggt gaccaccgtg     720

aaggtgccta tgatgaagcg tttaggcatg tttaacatcc agcactgtaa gaagctgtcc     780

agctgggtgc tgctgatgaa atacctgggc aatgccaccg ccatcttctt cctgcctgat     840

gaggggaaac tacagcacct ggaaaatgaa ctcacccacg atatcatcac caagttcctg     900

gaaaatgaag acagaaggtc tgccagctta catttaccca aactgtccat tactggaacc     960

tatgatctga agagcgtcct gggtcaactg ggcatcacta aggtcttcag caatggggct    1020

gacctctccg gggtcacaga ggaggcaccc ctgaagctct ccaaggccgt gcataaggct    1080

gtgctgacca tcgacgagaa agggactgaa gctgctgggg ccatgttttt agaggccata    1140

cccatgtcta tcccccccga ggtcaagttc aacaaaccct ttgtcttctt aatgattgaa    1200

caaaatacca agtctcccct cttcatggga aaagtggtga atcccaccca aaaataa       1257

<210>  2
<211>  1254
<212>  DNA
<213>  Homo sapiens

<400>  2
atgcccagca gcgtgtcctg gggaattctg ctgctggccg gcctgtgttg tctggtgcct      60

gtgtctctgg ccgaggaccc tcagggggat gccgcccaga aaaccgatac cagccaccac     120

gaccaggacc accccacctt caacaagatc acccccaacc tggccgagtt cgccttcagc     180

ctgtacagac agctggccca ccagagcaac agcaccaaca tctttttcag ccccgtgtct     240

atcgccaccg ccttcgccat gctgagcctg ggcacaaagg ccgacaccca cgacgagatc     300

ctggaaggcc tgaacttcaa cctgaccgag atccccgagg cccagatcca cgagggcttc     360

caggaactgc tgcggaccct gaaccagccc gatagccagc tgcagctgac aaccggcaac     420

ggcctgtttc tgagcgaggg actgaagctg gtggacaagt ttctggaaga tgtgaagaag     480

ctgtatcaca gcgaggcctt caccgtgaac ttcggcgaca ccgaggaagc caagaagcag     540

atcaacgact acgtggaaaa gggcacccag ggcaagatcg tggacctcgt gaaagagctg     600

gaccgggaca ccgtgttcgc cctcgtgaac tacatcttct tcaagggcaa gtgggagcgg     660

cccttcgaag tgaaggacac agaggaagag gactttcacg tggaccaagt gaccaccgtg     720

aaggtgccca tgatgaagag actgggcatg ttcaacatcc agcactgcaa gaaactgagc     780

agctgggtgc tgctgatgaa gtacctgggc aacgctaccg ccatattctt tctgcccgac     840

gagggcaagc tgcagcacct ggaaaacgag ctgacccacg acatcatcac caaatttctg     900

gaaaatgagg accggcggag cgccagcctg catctgccta agctgtctat caccggcacc     960

tacgacctga agtccgtgct gggacagctg ggcatcacca aggtgttcag caacggcgcc    1020

gatctgagcg gcgtgacaga agaggcccct ctgaagctgt ccaaggccgt gcacaaagcc    1080

gtgctgacca tcgacgagaa gggcaccgaa gccgctggcg ccatgtttct ggaagccatc    1140

cccatgagca tcccccctga agtgaagttc aacaagccct tcgtgttcct gatgatcgag    1200

cagaacacca agagccccct gttcatgggc aaggtcgtga accccaccca gaaa          1254

<210>  3
<211>  418
<212>  PRT
<213>  Homo sapiens

<400>  3

## Description

The present invention is in the field of compositions and dispensing systems for a pharmaceutical agent for the treatment of a viral infection in a subject.

The invention relates to a pharmaceutical composition comprising extra-cellular vesicles isolated from mesenchymal stem cells (MSCs) and Alpha-1 antitrypsin (AAT) protein, for use as a medicament in the treatment and/or prevention of a viral infection in a human subject. The invention further relates to the composition for topical and/or local administration to a mucosal surface of the respiratory tract and/or in the oral cavity of the subject. In another aspect the invention relates to a delivery system and corresponding formulations for dispensing the pharmaceutical composition.

### BACKGROUND OF THE INVENTION

Mesenchymal stem cells (MSCs) are cells of non-haematopoietic origin that reside in the bone marrow and other tissues. MSCs are commonly considered to be multipotent adult progenitor cells that have the ability to differentiate into a limited number of cell lineages, such as osteoblasts, chondrocytes, and adipocytes. Studies have been conducted on the use of MSCs as a therapeutic entity based on this capacity to differentiate directly into these end-stage phenotypes, including the use of MSCs to promote or augment bone repair and for the repair of cartilage defects (Vilquin and Rosset, Regenerative Medicine 2006: 1, 4 , p 589, and Veronesi et al, Stem Cells and Development 2013 ; 22, p 18 1). The isolation and cultivation of MSCs for a number of therapeutic indications has been described and represents a promising approach towards treating inflammation-associated disorders (for example WO 2010/119039).

MSCs interact with most of the cell types of the innate and acquired immune system, including B cells, T cells, dendritic cells (DCs), natural killer (NK) cells, neutrophils, and macrophages, moderating their response to pathogens. MSCs also play a role in the control of tissue inflammation. The therapeutic effects of MSCs have largely been attributed to their secretion of immunomodulatory and regenerative factors, and some of the effects may be mediated through host phagocytic cells which clear administered MSCs and in the process adapt an immunoregulatory and regeneration supporting function.

In response to inflammatory factors such as Interferon (IFNg) and Tumour Necrosis Factor (TNFα) secreted by activated immune cells and tissue cells, MSCs can adopt an immunoregulatory phenotype. They increase the expression of anti-inflammatory factors including programmed death ligand 1 and prostaglandin E2 and inhibit immune cell activity and proliferation through metabolic regulation, such as via indol-amine 2,3-dioxygenase-dependent catabolism of tryptophan. MSCs also express ATPases and possess ectonucleotidase activity through CD73 expression, through which they have the capacity to deplete ATP. The immunomodulatory effects of MSCs may also be triggered further by the activation of TLR receptor in MSCs, which is stimulated by pathogen-associated molecules such as LPS. Importantly, MSCs do not have an ACE2 receptor, which makes them resistant to SARS-CoV-2 infection (International Society for Infectious Diseases, international journal of infectious disease, 2020, p433).

MSCs appear to exert therapeutic effects through the secretion of factors to reduce cellular injury and enhance repair. The therapeutic effects of MSCs may be mediated by paracrine factors including extra-cellular vesicles such as exosomes, which are nanometer-sized membrane-bound vesicles, with functions as mediators of cell-cell communication. MSC-derived extra-cellular vesicles contain cytokines and growth factors, signaling lipids, mRNAs, and regulatory miRNAs. Increasing evidence suggests that MSC-derived exosomes might represent a novel cell-free therapy with compelling advantages over parent MSCs such as no risk of tumor formation and lower immunogenicity. Nonetheless, the exact mechanism of in vivo action of exogenously administered exosomes, their biodistribution, pharmacokinetics, and possibility of targeted delivery are not fully elucidated (Yin, et al. Biomark Res 7, 8 (2019).

Severe acute respiratory syndrome (SARS) is a Coronavirus (CoV) mediated respiratory disease which was first observed in 2002. Based on scientific reports it is assumed that all human CoVs may be of zoonotic origin. Once a human becomes infected, the virus can quickly spread via droplet transmission and close contact between humans, leading to epidemic scenarios or even to a pandemic. As one example, "Coronavirus Disease 2019" (COVID-19) is caused by the pathogenic corona virus SARS-CoV-2. Beginning in December 2019, the virus spread globally within a few weeks and led to an international health emergency. The worldwide pandemic poses huge challenges to health systems and leads additionally to restrictions in social life and weakening of global market economies. Since no effective therapy is available to date, and the disease is associated with high morbidity and mortality, there is a great need for therapeutic interventions for those who are ill as well as for prophylactic measures to contain outbreaks.

Recent reports have now investigated the potential of using exosomes derived from bone marrow mesenchymal stem cells as a treatment for severe COVID-19. Since the immune system is at the hub of the infection, it is essential to regulate the balance of immune responses in order to prevent an exaggerated and potentially deleterious immune response that can subsequently result in multiorgan damage. MSCs and MSC exosomes have immunomodulatory functions, they can affect immune cells by inducing anti-inflammatory macrophages, regulatory T and B cells, and regulatory dendritic cells, and can inactivate T cells, and could therefore be used as therapy to balance the immune reactions of patients with acute COVID-19 (Tsuchiya et al, Inflamm Regen. 2020; 40:14, Jayaramayya et al, BMB Repv. 53(8); 2020, Sengupta et al, Stem Cells Dev. June 15, 2020; 29(12): 747-754).

Alpha-1 antitrypsin (also known as A1AT, AAT, PI, SERPINA1) is a ∼52kDa glycoprotein that is one of the most abundant endogenous serine protease inhibitors (SERPIN superfamily). AAT is considered to be an acute phase protein, whereby AAT concentration can increase many fold upon acute inflammation.

Although known primarily for its anti-protease and anti-inflammatory activities, studies conducted over the past decade have cumulatively demonstrated that AAT is also an immune-modulator and a cytoprotective molecule. As such, microenvironments enriched with AAT were shown to contain reduced levels of pro-inflammatory cytokines, such as IL-1, IL-6, and TNF-α, and increased levels of anti-inflammatory mediators such as IL-1 receptor antagonist and IL-10. This phenomenon was also depicted in human PBMC in vitro studies, as well as in samples obtained from cystic fibrosis patients who received inhaled AAT. Concomitantly, AAT has been demonstrated to directly bind IL-8, and danger-associated molecular pattern molecules (DAMPs), such as extracellular HSP70 and gp96, which otherwise act as adjuvants to the associated immune responses (Lior et al., Expert Opinion on Therapeutic Patents, 2016).

Surprisingly, the anti-inflammatory qualities of AAT still allow innate immune cells to respond to authentic threats; macrophages readily phagocytose bacteria, neutrophils decontaminate infected sites, and antigen-loaded dendritic cells migrate to draining lymph nodes. T lymphocytes, on the other hand, respond to an AAT-rich environment indirectly, pending stimulation from innate immunocytes. For example, AAT has been shown to elicit semi-mature antigen presenting cells that favour the expansion of protective regulatory T cells. B lymphocytes, which belong to both the innate and adaptive immune system, appear to exert a modified response in the presence of AAT, in the form of diminished isotype switching, which results in enhanced protective IgM production. It has been suggested that the reduction in bacterial burden, as depicted in CF patients for instance, during treatment with AAT may relate to enhanced anti-pathogen immune responses; at the same time, local tissues are spared from inappropriate excessive injury that might promote deleterious adaptive responses by elevating the levels of local DAMPs (Lior et al., Expert Opinion on Therapeutic Patents, 2016).

Alpha 1-antitrypsin deficiency (AATD, or a 1-antitrypsin deficiency, or A1AD) is a medical disorder that causes defective production of alpha 1-antitrypsin (A1AT), leading to decreased A1AT activity in the blood and lungs, and deposition of excessive abnormal A1AT protein in liver cells. There are more than 75 known different mutations in SERPINA1, whereby 90% of the alpha 1-antitrypsin deficiency cases are due to a PI Z missense mutation: Glu342Lys. Deficiency of AAT leads to a chronic uninhibited tissue breakdown, whereby neutrophil elastase is free to break down alveolar interstitial elastin resulting in respiratory complications such as airway inflammation and emphysema. According to a report of Vianello in May 2020, the most common hereditary disorder in adults, AATD was found to have the highest prevalence rates in northern regions of Italy and to decrease notably from north to south. Recent data from the Italian Registry for AATD, showed a geographic distribution of confirmed cases very similar to the one reported for SARS-CoV-2 infection, with 47% of the total amount registered in the Lombardia region (237 out 508).

Reports have indicated that the production of AAT is increased in COVID-19, but this anti-inflammatory response is overwhelmed in severe illness (McElvaney et al, Am J Respir Crit Care Med. 2020 Sep 15;202(6):812-821). The entry of SARS-CoV-2 into an infected cell is mediated by binding of the viral spike protein via its receptor binding domain (RBD) to the human angiotensin converting enzyme-2 (ACE2) target receptor. For example, blocking this interaction by human antibodies leads to a neutralization of the virus in patients and thus to a healing of the infection. Further work has also shown that entry of SARS-CoV-2 is facilitated by endogenous and exogenous proteases. These proteases proteolytically activate the SARS-CoV-2 spike glycoprotein and are key modulators of virus tropism. AAT has been identified as an abundant serum protease inhibitor that potently restricts protease-mediated entry of SARS-CoV-2. AAT inhibition of protease-mediated SARS-CoV-2 entry in vitro occurs at concentrations below what is present in serum and bronchoalveolar tissues, suggesting that AAT effects are physiologically relevant (Oguntuyo et al, bioRxiv. Aug 15; 2020).

Despite these advances, to the knowledge of the inventors, there has been no suggestion in the art until now to employ extra-cellular vesicles from MSCs to prevent viral infection. Previous reports have focused on the treatment of acute COVID illness and modulation of the unwanted overreaction of the immune response using MSC exosomes. Furthermore, to the knowledge of the inventors, there has been no suggestion in the art to combine extra-cellular vesicles from MSCs with AAT administration, in order to treat and/or prevent viral infection.

### SUMMARY OF THE INVENTION

In light of the prior art the technical problem underlying the present inventions is to provide alternative and/or improved means for the treatment of viral infection.

This problem is solved by the features of the independent claims. Preferred embodiments of the present invention are provided by dependent claims.

The invention therefore relates to a pharmaceutical composition comprising extra-cellular vesicles isolated from mesenchymal stem cells (MSCs) and Alpha-1 antitrypsin (AAT) protein, for use as a medicament in the treatment and/or prevention of a viral infection in a human subject.

AAT has systematic anti-inflammatory effects and a local activity in lung tissue. Advantageously, the anti-inflammatory qualities of AAT still allow innate immune cells to respond to authentic threats; macrophages readily phagocytose bacteria, neutrophils decontaminate infected sites, and antigen-loaded dendritic cells migrate to draining lymph nodes. AAT therefore is capable of exerting multiple effects, including reducing inflammation and an unwanted cytokine storm resulting from viral infection, while still enabling effective immune responses against pathogens, and AAT also appears capable of actively inhibiting viral infection, in particular by SARS-CoV-2. Proteases are known to activate the SARS-CoV-2 spike glycoprotein and are therefore modulators of virus tropism. AAT is a serum protease inhibitor that can restrict protease-mediated entry of SARS-CoV-2.

MSCs appear to exert anti-inflammatory and immunoregulatory functions, promote the regeneration of damaged tissues and inhibit tissue fibrosis. The immunomodulatory effects of MSCs involves both direct and indirect effects on the host immune cells. The therapeutic effect of MSCs is mediated in part by the secretion of factors including extra-cellular vesicles, such as exosomes, from MSCs, and the potential of using MSC exosomes to modulate the immune response in SARS-CoV-2 infection appears promising.

The combination of extra-cellular vesicles isolated from mesenchymal stem cells (MSCs) and Alpha-1 antitrypsin (AAT) protein therefore represents a beneficial approach towards achieving the combined effects of immune modulation during infection and restriction of viral infection of target cells.

Surprisingly, the combination of extra-cellular vesicles isolated from MSCs with AAT protein leads to an enhanced prevention of viral infection in target cells and/or an enhanced immune response against infection, which is greater than the sum of the effects achieved by either agent when administered alone. In some embodiments, the combined effect of extra-cellular vesicles isolated from MSCs with AAT protein leads to a synergistic effect in the prevention of viral infection in target cells and/or an enhanced immune response against infection. In some embodiments, the combined effect of extra-cellular vesicles isolated from MSCs with AAT protein represents a previously unsuggested technical effect in immune modulation during infection and restriction of viral infection of target cells. In some embodiments, this preferably synergistic effect is a novel approach towards preventing viral infection.

According to the knowledge of the inventors, a pharmaceutical composition comprising extra-cellular vesicles isolated from mesenchymal stem cells and Alpha-1 antitrypsin protein has not been previously proposed for the treatment/prevention of a viral infection in a human subject. It was particularly surprising that a pharmaceutical composition comprising extra-cellular vesicles isolated from mesenchymal stem cells and Alpha-1 antitrypsin protein is capable of treating and/or preventing a viral infection in a human subject.

In one embodiment, the AAT protein is an isolated AAT protein, added to the composition. For example, the AAT may be recombinant AAT protein, or may be purified AAT protein, for example purified from human plasma, and then added to a composition, ultimately for combination with the extra-cellular vesicles. In one embodiment, the AAT protein may be produced in the MSCs from which the extra-cellular vesicles are isolated. In one embodiment, the AAT protein is endogenous to the MSCs from which the extra-cellular vesicles are obtained. This means, the AAT protein is expressed from the original gene and promoter (e.g. from an unmodified MSC).

In one embodiment, the AAT protein is exogenous protein. In one embodiment, the AAT is exogenous to the MSC from which the extra-cellular vesicles are obtained. For example, an AAT-encoding transgene may be expressed from the MSC, for example to obtain large amounts of AAT expression, such that the extra-cellular vesicles comprise AAT, preferably in relatively large amounts, for example in larger amounts, than the amount of AAT in the extra-cellular vesicles obtained from an unmodified MSC.

In one embodiment, the invention relates to a pharmaceutical composition as described herein for use as a medicament in the treatment and/or prevention of a viral infection in a human subject, wherein the AAT protein is expressed in the mesenchymal stem cells (MSCs) from which the extra-cellular vesicles are isolated, wherein the MSCs are genetically modified to express exogenous Alpha-1 antitrypsin (AAT).

In this embodiment, the AAT is provided to the extra-cellular vesicles, either within the extra-cellular vesicles or associated with the extra-cellular vesicles, by expression of preferably transgenic (exogenous) AAT in the MSCs from which the extra-cellular vesicles are obtained. The AAT produced by transgenic expression in the modified MSCs surprisingly and beneficially is present in the extra-cellular vesicles at a significant amount, suitable to enhance the anti-viral effect. In some embodiments, the exogenous AAT is produced from the endogenous coding region/gene, but by expression through a modified promoter with greater expression levels.

In some embodiments, the extra-cellular vesicles and alpha-1 antitrypsin (AAT) are both obtained from mesenchymal stem cells (MSCs) which are genetically modified to express exogenous Alpha-1 antitrypsin (AAT). This combination provides a surprising effect of treating/preventing viral infection of a human subject.

In one embodiment, the invention relates to a pharmaceutical composition as described herein for use as a medicament in the treatment and/or prevention of a viral infection in a human subject, wherein the composition is enriched with Alpha-1 antitrypsin (AAT) protein, preferably enriched with isolated recombinant AAT protein.

Isolated AAT protein can therefore be added to the extra-cellular vesicles from MSCs, either from extra-cellular vesicles of unmodified MSCs or to extra-cellular vesicles from modified AATs expressing transgenic AAT.

AAT can be recombinantly generated in a variety of systems including bacteria, insect cells, or mammalian cells. Recombinantly generated AAT possesses post-translational modifications, preferably distinct glycosylation, more preferably N-glycosylation and/or O-glycosylation. The changes in the glycosylation pattern of AAT affect primarily the rate of protein degradation both in endoplasmic reticulum and in plasma, as well as the rate by which the protein aggregates. These factors extend the plasma half-life of AAT protein from 7 min to 1 day, preferably 2-days, more preferably 3 to 4 days. Therefore, in some embodiments, AAT is preferentially optimized through the correct set of post-translational modifications, obtained either by expression as endogenous protein, and isolation, or obtained through expression in an appropriate recombinant system.

In some embodiments, AAT is extracted from human plasma. AAT is in some embodiments extracted from pooled human plasma using AAT-enriched plasma preparations.

Mesenchymal stem cells interact with most of the cell types of the innate and acquired immune system, including B cells, T cells, dendritic cells (DCs), natural killer (NK) cells, neutrophils, and macrophages, moderating their response to pathogens. MSCs also play a role in the control of tissue inflammation. The therapeutic effects of MSCs have largely been attributed to their secretion of immunomodulatory and regenerative factors, and some of the effects may be mediated through host phagocytic cells which clear administered MSCs and in the process adapt an immuno- regulatory and regeneration supporting function. Considering that the extra-cellular vesicles of MSCs can mediate these functions, the vesicles may be obtained from MSCs, isolated and administered in combination with AAT, either expressed from the AAT-genetically modified cells themselves or from AAT added to isolated MSCs (enrichment).

In preferred embodiments, recombinant AAT expressed from mesenchymal stem cells has the beneficial effects of an immunomodulatory function, homes to lung tissue, reduces cytokine storms, prevents entry of SARS-CoV-2 into host cell and/or prevents damage to lung tissue.

It was unexpected that a pharmaceutical composition described herein enriched with AAT, preferably enriched with isolated recombinant AAT protein could be used as a medicament in the treatment and/or prevention of a viral infection.

In one embodiment, the invention relates to a pharmaceutical composition as described herein for use as a medicament in the treatment and/or prevention of a viral infection, wherein the extra-cellular vesicles are exosomes and/or microvesicles.

In one embodiment, the invention relates to a pharmaceutical composition as described herein for use as a medicament in the treatment and/or prevention of a viral infection, wherein the extra-cellular vesicles comprise AAT protein, preferably expressed form the modified MSCs, and one or more biological factors selected from the group consisting of cytokines, such as IFN-γ, IL-8, IL-10 and TGF-β, HLA-G, cholesterol, ceramide, phosphoglycerides, saturated fatty-acyl chains, prostaglandins and nucleic acids, such as RNA, preferably miRNA, siRNA and shRNA.

In one embodiment, the invention relates to a pharmaceutical composition as described herein for use as a medicament in the treatment and/or prevention of a viral infection, wherein the extra-cellular vesicles have an average diameter of 1-500nm, preferably between about 10 to 200 nm, more preferably between 20 to 150nm.

In one embodiment, the invention relates to a pharmaceutical composition as described herein for use as a medicament in the treatment and/or prevention of a viral infection, wherein the concentration of the extra-cellular vesicles is 0.01-100 mg/ml, preferably 0.1-10 mg/ml, more preferably 1-5 mg/ml.

Exosomes are extracellular vesicles whose membranous structures measure between 1 and 500 nm, preferably between about 10 to 200 nm, more preferably between 20 to 150nm. Exosomes contain cytokines, such as IFN-γ, IL-8, IL-10 and TGF-β, HLA-G, cholesterol, ceramide, phosphoglycerides, saturated fatty-acyl chains, prostaglandins and nucleic acids, such as RNA, preferably miRNA, siRNA and shRNA. Physiologically, exosomes assist in the intercellular transport of protein and RNA. Immunologically, exosomes can exhibit an antigen-presenting capability. Exosomes were likewise found to be associated with the pathophysiology of cardiovascular, renal, neurological, and ocular diseases. In addition, exosomes may play a major role in cancer metastasis. It is surprising that exosomes isolated from MSC could be used as a medicament in treatment and/or prevent a viral infection, and furthermore do not show unwanted side effects with respect to pathophysiological effects.

In some embodiments, the invention relates to a pharmaceutical composition as described herein for use as a medicament in the treatment and/or prevention of a viral infection, wherein the extra-cellular vesicles are exosomes, which are obtainable by a method comprising the following steps:
a) Providing a cell culture supernatant comprising exosomes from mesenchymal stem cells (MSCs), optionally genetically modified to express exogenous Alpha-1 antitrypsin (AAT); and
b) Isolating the exosomes by removing the cells and/or cell debris, preferably by centrifugation, high-performance liquid chromatography (HPLC) and/or ultrafiltration.

The exosomes in some embodiments are obtainable by a method further comprising determining the protein concentration of exosomes isolated by removing the cells and/or cell debris, preferably by centrifugation, high-performance liquid chromatography (HPLC) and/or ultrafiltration.

Due to the extremely small size and scarcity of exosomes in living samples, exosomes are isolated by removing the cells and/or cell debris, preferably by centrifugation, high-performance liquid chromatography (HPLC) and/or ultrafiltration, using techniques established in the art. These isolation methods are preferably achieved by means of sucrose density gradient ultracentrifugation, preferably immunoaffinity and/or microfluidic system approaches which are efficient.

In one embodiment, the invention relates to a pharmaceutical composition as described herein for use as a medicament in the treatment and/or prevention of a viral infection, wherein the mesenchymal stem cells (MSCs) are selected from human, murine, rat mesenchymal stem cells.

In some embodiments, the invention relates to a pharmaceutical composition as described herein for use as a medicament in the treatment and/or prevention of a viral infection, wherein said MSCs are selected from human MSCs derived from induced pluripotent stem cells (iPSCs), umbilical cord blood, umbilical cord tissue, placenta, bone marrow, bone or adipose tissue and MSCs that have been cultured in platelet lysate.

In one embodiment the invention relates to a pharmaceutical composition as described herein for use as a medicament in the treatment and/or prevention of a viral infection, wherein said composition is administered topically and/or locally to a mucosal surface of the respiratory tract and/or oral cavity of the subject.

It was surprising that the pharmaceutical composition described herein could be administered topically and/or locally to a mucosal surface of the respiratory tract and/or cavity of the subject. The advantages are the medicament could be applied directly to the skin. There are reduced side effects and toxicity to other organs compared to systemic medications. It was also surprising that the topical or local administration did not lead to significant AAT and/or vesicle degradation. Both the formulations comprising the combination of AAT and extra-cellular vesicles, prior to administration, and the composition after administration, appear sufficiently stable in order to achieve a therapeutic and/or prophylactic effect.

In one embodiment, the invention relates to a pharmaceutical composition as described herein for use as a medicament in the treatment and/or prevention of a viral infection, wherein the composition is suitable for administration to the upper or lower respiratory tract, the lungs, nasal cavity, oral cavity and/or throat of a subject.

This leads to a local efficacy within the airway of respiration including, upper or lower respiratory tract, the lungs, nasal cavity, oral cavity and/or throat of a subject. Unwanted systemic effects are minimised, as the delivered drug acts with maximum pulmonary specificity combined with a rapid onset and efficacious duration of action.

In some embodiments, the invention relates to a pharmaceutical composition as described herein for use as a medicament in the treatment and/or prevention of a viral infection, wherein said composition is in liquid or powder form and is suitable for spraying in the nasal and/or oral cavity of a subject and/or for inhalation.

The composition in powder form has the advantage of high stability and avoids potential preservatives that may otherwise be required in liquid formulations. The composition in liquid or suspension form is advantageous as it provides high local drug (active agen/here the vesicles and/or AAT) concentration, rapid onset of drug action, superior respiratory selectivity, reduced systemic drug exposure and reduced side effects.

In some embodiments, the composition could also be in an aerosol form which is either a solution containing the active agent, suspensions of solid drug particles in a gas or a dry powder. An aerosol has three advantageous factors, i.e. smaller particle size, lower plume velocity and lower temperature. These factors may decrease upper airway impaction and increase airway deposition of particles, particularly to the small airways, compared with the same drug administered from a chlorofluorocarbon-driven pressurised metered-dose inhaler.

In one embodiment, the invention relates to a pharmaceutical composition as described herein for use as a medicament in the treatment of a viral infection wherein said composition is administered by introducing a therapeutically effective amount of the composition to the blood stream, eyes, nasal cavity, oral cavity, throat and/or lung.

In another embodiment, the invention relates to a pharmaceutical composition as described herein for use as a medicament in the treatment of a viral infection wherein said composition is administered by intravenous injection, subcutaneous injection, intraperitoneal injection and/or inhalation, preferably inhalation.

In some embodiments, the invention relates to a pharmaceutical composition as described herein for use as a medicament in the treatment of medical condition further associated with cardiovascular disease, kidney injury, immune disease, tumour, neurological disease.

In one embodiment, the invention relates to a pharmaceutical composition as described herein for use as a medicament in the treatment and/or prevention of a viral infection, wherein the subject is suspected of having a viral infection and/or has been in proximity and/or in contact with an individual with a viral infection.

The pharmaceutical composition as described herein is intended for a prophylactic use (or use to reduce risk) to prevent (or reduce the risk of) the onset of a disease resulting from a viral infection in subject who has symptoms of viral infection and/or has been in proximity and/or in contact with an individual with a viral infection. Furthermore, the pharmaceutical composition as described herein has been proposed for the first time to be used as a medicament for treating a viral infection.

In some embodiments, the invention relates to the pharmaceutical composition as described herein for use as a medicament in the treatment of a medical condition associated with fever, cough, tiredness, inflammation, aching limbs, sore throat, itchy throat, diarrhoea, conjunctivitis, headaches, loss of the sense of taste or smell, discoloration on fingers or toes or skin rash, breathing difficulty, shortness of breath, pain or feeling of pressure in the chest area and/or loss of speech or movement ability. In some embodiments, the patient with or at risk of a viral infection has such symptoms.

In some embodiments the invention relates to the pharmaceutical composition as described herein for use as a medicament in the treatment of a medical condition associated further with pneumonia, viral sepsis, acute respiratory distress, kidney failure, cytokine release.

In one embodiment the invention relates to the pharmaceutical composition as described herein for use as a medicament in the treatment of medical condition wherein said medical condition results from a viral infection, preferably with an enveloped, positive-strand RNA virus.

In a preferred embodiment, the invention relates to a pharmaceutical composition as described herein for use as a medicament in the treatment and/or prevention of a viral infection, wherein the virus is from the family of coronaviridae, preferably a betacoronavirus, more preferably a SARS-CoV.

In a more preferred embodiment, the invention relates to a pharmaceutical composition as described herein for use as a medicament in the treatment and/or prevention (preferably prevention) of a viral infection, wherein the virus is SARS-CoV-2. In preferred embodiments, the invention is directed to treatment and/or prevention of SARS-CoV-2 infections by local and/or topical administration in the oral cavity and/or respiratory tract of a subject at risk of or with such a condition.

It appears that all three lethal zoonotic coronaviruses, MERS-CoV, SARS-CoV and SARS-CoV-2 induce excessive and aberrant host immune responses, which are associated with severe lung pathology leading to acute respiratory distress syndrome. SARS-CoV-2 enters the host cells via the cell surface angiotensin converting enzyme 2 (ACE2). An associated cytokine storm may play a role in pathogenesis. Proinflammatory cytokines and chemokines, including tumour necrosis factor (TNF)α, interleukin 1β (IL-1β), IL-6, granulocyte-colony stimulating factor, interferon gamma-induced protein-10, monocyte chemoattractant protein-1, and macrophage inflammatory proteins 1-α, were significantly elevated in COVID-19 patients.

It is a surprising effect that the composition as described herein achieves the effect of therapeutic interventions which can dampen the excess inflammation, thus preventing end organ damage and long-term functional disability in those who survive severe disease, but at the same time enable an effective immune response against the pathogen.

In another embodiment the invention relates to a pharmaceutical composition for use as described herein wherein the composition prevents transmission of virus, preferably SARS-CoV, more preferably SARS-CoV-2.

The transmembrane serine protease TMPRSS2 is indispensable for S protein priming of the MERS, SARS-CoV, and SARS-CoV2 coronaviruses, a process that is necessary for entry of the virus into host cells. Therefore, inhibiting TMPRSS2 holds promise as an approach toward preventing transmission of coronaviruses. It is surprising to find that Alpha 1 Antitrypsin is an Inhibitor of the SARS-CoV2-Priming Protease TMPRSS2.

In another embodiment the invention relates to a pharmaceutical composition as described herein for use as a medicament in combination with an existing treatment or as pre-treatment for a viral infection, preferably an infection of SARS-CoV-2.

In a further aspect, the invention relates to a delivery system for delivering a pharmaceutical composition as described herein for use as a medicament in the treatment and/or prevention of a viral infection, the delivery system comprising: a) a pharmaceutical composition according to any one of the preceding claims, with one or more pharmaceutical excipient(s), and b) a dispenser.

In a preferred embodiment, the invention relates to a delivery system for delivering a pharmaceutical composition as described herein for use as a medicament in the treatment and/or prevention of a viral infection, wherein the composition is in liquid or powder form and the dispenser is suitable for spraying the composition in the nasal and/or oral cavity of a subject and/or for inhalation of the composition.

In one embodiment, the composition is formulated for cryopreservation without significant functional loss of the isolated extra-cellular vesicles.

In one aspect of the invention, the composition comprises liquid formulations. Liquid formulations currently dominate the nasal drug market because they are considered convenient particularly for topical indications where humidification counteracts the dryness and crusting, which could otherwise accompany chronic nasal disease. There are a variety of liquid formulation devices on the market such as Rhinyle catheter from Ferring, unit-dose pipettes GlaxoSmithKline, etc.

In another aspect of the invention, the composition comprises powder formulations. Powder formulations can offer advantages including greater stability and potential avoiding use of preservatives otherwise used in liquid compositions. Powders tend to stick to the moist surface of the nasal mucosa before being dissolved and cleared. There are a variety of dry powder devices on the market such as Turbohaler from AstraZeneca, Diskus/Accuhaler from GlaxoSmithKline, Genuair from Almirall, Airmax from Norton Healthcare etc.

In one aspect of the invention, the composition comprises suspension formulations. Inhalation solution and suspension drug products are typically aqueous-based formulations that contain therapeutically active ingredients and can also contain additional excipients. The advantages of the suspension formulations are similar as with liquid formulations.

In another aspect of the invention, the composition comprises an aerosol form. An aerosol is a suspension of liquid and/or solid particles produced by an aerosol generator, such as the small-volume nebulizer (SVN), the pressurized metered-dose inhaler (pMDI), or the dry-powder inhaler (DPI). Aerosol deposition is a process of aerosol particles depositing on absorbing surfaces.

The major mechanisms of aerosol deposition include, without limitation, inertial impaction, gravitational sedimentation (settling), and diffusion. Inertial impaction occurs with larger (>3 µm), fast-moving particles. Gravitational settling is a function of particle mass and time, with the rate of settling proportional to particle size and mass. Diffusion occurs with particles smaller than 1 µm. These mechanisms come into play as aerosol particles are inhaled orally or through the nose. Larger particles (> 10 µ) generally reach the oropharynx, largely by inertial impaction; particles of 5-10 µm generally reach the proximal generations of the lower respiratory tract, and particles of 1-5 µm reach to the lung periphery.

Particle size plays an important role in lung deposition, along with particle velocity and settling time. As particle size increases above 3 µm, aerosol deposition shifts from the periphery of the lung to the conducting airways. Oropharyngeal deposition increases as particle size increases above 6 µm. Exhaled loss is high with very small particles of 1 µm or less. Consequently, particle sizes of 1-5 µm are often preferred for reaching the lung periphery, whereas 5-10 µm particles deposit mostly in the conducting airways, and 10-100 µm particles deposit mostly in the nose.

In embodiments of the invention, the above-mentioned aerosol sozes are intended for formulation and dispensing the composition of the present invention, thereby enabling either one particular part of the respiratory tract to be targeted or by combining aerosols of different sizes to achieve distribution of the active agent throughout various regions of the oral cavity and/or respiratory tract.

The advantages of an aerosolized drug are that 1) aerosol doses are generally smaller than systemic doses; 2) Onset of effect with inhaled drugs is typically faster than with traditional oral dosing; 3) drug is delivered directly to the lungs with minimal systemic exposure; 4) systemic side effects are less frequent and severe with inhalation when compared to systemic delivery; and 5) inhaled drug therapy is less painful than injection and is relatively comfortable. Currently available aerosol drug formulation with corresponding inhaler device are for example AccuNeb ^{®}, Xopenex^{®}, and ProAir HFA^{®}.

These forms of composition could be made to be suitable for spraying and/or inhalation in nasal cavity and/oral cavity, using the composition of the present invention.

In preferred embodiments, a dispenser is used to convert the pharmaceutical composition into liquid, or powder or suspension or aerosol, which is suitable for inhalation and/or spraying so that the particles of the medication arrive directly into the airway, lungs or other regions of the respiratory tract and/or oral and/or nasal cavity. A dispenser is preferably selected from the group of small-volume nebulizers, pressurized metered-dose inhalers, metered-dose inhaler accessory devices, dry powder inhalers, nasal sprays and oral cavity sprays.

In one embodiment of the invention, the dispenser is a small-volume nebulizer. The advantages are 1) able to aerosolize any drug solution; 2) able to aerosolize a drug mixture (> 1 drug), if drugs are compatible; 3) minimal patient cooperation or coordination needed; 4) useful in very young, very old, debilitated, or distressed patients; 5) drug concentrations and dose can be modified; 6) variability in performance characteristics among different types, brands and models; and 7) normal breathing patterns can be used, and an inspiratory pause is not required for efficacy.

In one embodiment of the invention, the dispenser is a pressurized metered-dose inhaler. The advantages are that the dispenser is portable, light, compact, has multiple dose convenience, a short treatment time, reproducible emitted doses, no drug preparation is required, and the formulation is difficult to contaminate.

In another aspect of the invention, the dispenser is a metered-dose inhaler accessory device. The advantages are reduced oropharyngeal drug impaction and loss, increased inhaled drug by two to four times than the pMDI (Pressurized metered dose inhaler, or puffer) alone, allowing use of the pMDI during acute airflow obstruction with dyspnea, no drug preparation required, simplifying coordination of pMDI actuation and inhalation, and helping reduce local and systemic side effects.

In one embodiment of the invention, the dispenser is a dry-powder inhaler. The advantages are small and portable, built-in dose, propellant free, breath-actuated, short preparation and administration time.

In a further aspect, the invention relates to a pharmaceutical combination. The features of the composition as described herein may be used to describe the combination.

In a preferred embodiment, the invention relates to a pharmaceutical combination, comprising:
(a.) a therapeutically effective amount of extra-cellular vesicles, preferably exosomes, from MSCs, and
(b.) a therapeutically effective amount of AAT.

The invention also relates to the combination for use in the treatment of a viral infection, such as Coronavirus infection, and/or for the treatment and/or prophylaxis of SARS Coronavirus-associated respiratory disease, and corresponding methods of treatment. The invention also relates to the combined administration of a therapeutically effective amount of extra-cellular vesicles, preferably exosomes, from MSCs and a therapeutically effective amount of AAT in such treatment.

The combination of the invention therefore relates to a previously undescribed and advantageous combination, preferably characterised by a synergistic effect between the two active agents.

A skilled person would not have expected these two agents effective against viral infection or disease to interact beyond a cumulative effect. Even if reduced virus growth and/or infection, achieved by the extra-cellular vesicles and AAT when each is administered alone, has been derived from the art, a synergistic effect could not have been predicted based on the expectation of a skilled person. Typically, targeting two mechanisms with anti-viral effects would not typically lead to synergistic effects, but to no additional or negligible additive effects. Without being bound by theory, a second (combined) factor would typically lead to no additional or at most a small cumulative effect, as targeting two targets in an anti-viral effect or related effects would typically not lead to further quantitative effects, as the viral infection has already been inhibited by the first agent, such that by targeting another position in the same or a related anti-viral effect by a second agent would be expected to not have an additional effect, but merely reinforce the effect already achieved by the first agent. Contrary to expectations, a surprising and quantitative synergy has been achieved by the extra-cellular vesicles and AAT.

In some embodiments, the extra-cellular vesicles and AAT have relative amounts of 10000:1 to 1:10000 by weight, preferably 10:1 to 1:10 by weight, more preferably about 5:1 to 1:5 by weight.

Embodiments and features of the invention described with respect to the composition comprising extra-cellular vesicles from MSCs and AAT for the treatment and/or prevention of a viral infection and a delivery system are considered to be disclosed with respect to each and every other aspect of the disclosure, such that features characterizing the composition, combination, or delivery system, may be employed to characterize the composition, and vice-versa. The various aspects of the invention are unified by, benefit from, are based on and/or are linked by the common and surprising finding of a pharmaceutical composition comprising extra-cellular vesicles isolated from mesenchymal stem cells (MSCs) and Alpha-1 antitrypsin (AAT) protein, for use as a medicament in the treatment and/or prevention of a viral infection in a human subject.

### DETAILED DESCRIPTION OF THE INVENTION

All cited documents of the patent and non-patent literature are hereby incorporated by reference in their entirety.

### Mesenchymal stem cells:

As used herein, "mesenchymal stem cells" (also referred to in some embodiments as "mesenchymal cells" or "MSCs", and also known as mesenchymal stromal cells) are multipotent cells that can differentiate into connective tissue, bone, cartilage, and cells in the circulatory and lymphatic systems. Mesenchymal stem cells are found in the mesenchyme, the part of the embryonic mesoderm that consists of loosely packed, fusiform or stellate unspecialized cells. As used herein, mesenchymal stem cells include, without limitation, CD34-negative stem cells.

In one embodiment of the invention, the mesenchymal cells are fibroblast-like plastic adherent cells, defined in some embodiments as multipotent mesenchymal stromal cells and also include CD34-negative cells. For the avoidance of any doubt, the term mesenchymal stem cell encompasses multipotent mesenchymal stromal cells that also includes a subpopulation of mesenchymal cells, MSCs and their precursors, which subpopulation is made up of multipotent or pluripotent self-renewing cells capable of differentiation into multiple cell types in vivo.

As used herein, CD34-negative cell shall mean a cell lacking CD34, or expressing only negligible levels of CD34, on its surface. CD34-negative cells, and methods for isolating such cells, are described, for example, in Lange C. et al., "Accelerated and safe expansion of human mesenchymal stromal cells in animal serum-free medium for transplantation and regenerative medicine". J. Cell Physiol. 2007, Apr. 25.

Mesenchymal cells can be differentiated from hematopoietic stem cells (HSCs) by a number of indicators. For example, HSCs are known to float in culture and to not adhere to plastic surfaces. In contrast, mesenchymal cells adhere to plastic surfaces. The CD34-negative mesenchymal cells of the present invention are adherent in culture.

MSCs can be cultured using known protocols. For example, human MSCs derived from induced pluripotent stem cells (iPSCs), umbilical cord blood, umbilical cord tissue, placenta, bone marrow, bone or adipose tissue and MSCs can be cultured as adherent cells on plastic surfaces, in the presence of platelet lysate. As used herein, "platelet lysate" is a substitute supplement for fetal bovine serum (FBS) in experimental and clinical cell culture. It is obtained from human blood platelets after freeze/thaw cycle(s). The freeze/thaw cycle causes the platelets to lyse, releasing a large quantity of growth factors necessary for cell expansion. FBS-free cell culture media, e.g. with platelet lysate or chemically defined/ animal component free, are used for cell therapy or regenerative medicine. They are commercially available in GMP (good manufacturing practice)-quality which is generally basis for regulatory approval. Pooled human platelet lysate has been used as a culture supplement to promote MSC growth. Alternative MSC culturing procedures are also available and known to a skilled person.

### Genetically modified MSCs:

MSCs can be genetically modified, for example to express transgenic AAT, using techniques known in the art. In some embodiments, AAT-encoding nucleic acids are introduced into an MSC, preferably stably integrated, or stably maintained in the cell, and subsequently expressed from the modified MSC.

As used herein, "genetical modification" also called genetic engineering, genetic modification is a technique to change the characteristics of a plant, animal or micro-organism by transferring a piece of DNA obtained by either isolating and or copying the genetic material of interest using recombinant DNA methods or by artificially synthesising the DNA to a different organism. This is done through inserting them to the other organism. This technique has for example been used to develop mammalian cell, fungi and bacteria that produce medicines. There are a variety of genetic modification methods: microbal vectors, microprojectile bombardment, electroporation, microinjection, transponses/transponsable elements, cloning, transfection, retroviral vectors, knock-in and knock-out technology, marker-assisted selection

Techniques suitable for modifying MSC include, without limitation, the use of viral vectors, such as a retrovirus, lentivirus, adenovirus, adeno-associated virus, non-viral vectors (Liposomes and plasmids), or gene editing, such as CRISPR/Cas9, ZFNs and/or TALENs. Viral vectors are the most commonly used tool in the MSC genetic modification protocols, and it has been demonstrated that the high efficiency of viral transduction in these cells does not affect their immunophenotypic characteristics, as well as their potential for cell differentiation and secretion of bioactive molecules, which are preserved after genetic modification. In addition, viral transduction ensures stable and long-term transcription of the gene of interest and, consequently, a greater efficiency compared to other methods that do not use viral vectors for genetic modification of MSCs. The site-specific integration of genes can be achieved using tools such as Zinc Finger Nuclease (ZFN), Transcription Activator-Like Effector Nuclease (TALENS) or Clustered Regularly Interspaced Short Palindromic Repeats (CRISPR/Cas9), which are nucleases capable of recognizing and direct the integration of genes in a site-specific manner. These molecular-editing tools are also highly efficient and accurate and can be employed to improve safety and efficiency of gene insertion/expression.

Any given gene delivery method is encompassed by the invention and preferably relates to viral or non-viral vectors, as well as biological or chemical methods of transfection. The methods can yield either stable or transient gene expression in the system used.

Genetically modified viruses have been widely applied for the delivery of genes into stem cells. Preferred viral vectors for genetic modification of the MSCs described herein relate to retroviral vectors, in particular to gamma retroviral vectors. The gamma retrovirus (sometimes referred to as mammalian type C retroviruses) is a sister genus to the lentivirus clade, and is a member of the Orthoretrovirinae subfamily of the retrovirus family. The Murine leukemia virus (MLV or MuLV), the Feline leukemia virus (FeLV), the Xenotropic murine leukemia virus-related virus (XMRV) and the Gibbon ape leukemia virus (GALV) are members of the gamma retrovirus genus. A skilled person is aware of the techniques required for utilization of gamma retroviruses in genetic modification of MSCs. For example, the vectors described by Maetzig et al (Gammaretroviral vectors: biology, technology and application, 2001, Viruses Jun;3(6):677-713) or similar vectors may be employed. For example, the Murine Leukemia Virus (MLV), a simple gammaretrovirus, can be converted into an efficient vehicle of genetic therapeutics in the context of creating gamma retrovirus-modified MSCs and expression of a therapeutic transgene from said MSCs after delivery to a subject.

Adenoviruses may be applied, or RNA viruses such as Lentiviruses, or other retroviruses. Adenoviruses have been used to generate a series of vectors for gene transfer cellular engineering. The initial generation of adenovirus vectors were produced by deleting the EI gene (required for viral replication) generating a vector with a 4kb cloning capacity. An additional deletion of E3 (responsible for host immune response) allowed an 8kb cloning capacity. Further generations have been produced encompassing E2 and/or E4 deletions. Lentiviruses are members of Retroviridae family of viruses (M. Scherr et al., Gene transfer into hematopoietic stem cells using lentiviral vectors. Curr Gene Ther. 2002 Feb; 2(1):45-55). Lentivirus vectors are generated by deletion of the entire viral sequence with the exception of the LTRs and cis acting packaging signals. The resultant vectors have a cloning capacity of about 8 kb. One distinguishing feature of these vectors from retroviral vectors is their ability to transduce dividing and non-dividing cells as well as terminally differentiated cells.

Non-viral methods may also be employed, such as alternative strategies that include conventional plasmid transfer and the application of targeted gene integration through the use of integrase or transposase technologies. These represent approaches for vector transformation that have the advantage of being both efficient, and often site-specific in their integration. Physical methods to introduce vectors into cells are known to a skilled person. One example relates to electroporation, which relies on the use of brief, high voltage electric pulses which create transient pores in the membrane by overcoming its capacitance. One advantage of this method is that it can be utilized for both stable and transient gene expression in most cell types. Alternative methods relate to the use of liposomes or protein transduction domains. Appropriate methods are known to a skilled person and are not intended as limiting embodiments of the present invention.

In one embodiment, the exogenous AAT expressed from a transgenic AAT gene as a product of genetic modification of the MSCs, is controlled by a constitutive promoter, or by an inducible or conditional promoter, preferably a constitutive promoter.

As used herein, a constitutive promoter is an essentially unregulated promoter that allows for continual transcription of its associated gene. Constitutive promoters are preferred, as they enable high and consistent expression of AAT, thereby enriching the protein in the exosomes that are employed in the invention. In one embodiment the genetically modified mesenchymal stem cell as described herein is characterised in that the AAT-expressing promoter is an EFI alpha promoter, for example the EFIalphaS promoter, the PGK promoter, the CMV or SV40 viral promoters, the GAG promoter or the UB promoter.

As used herein, "nucleic acid" shall mean any nucleic acid molecule, including, without limitation, DNA, RNA and hybrids or modified variants thereof. An "exogenous nucleic acid" or "exogenous genetic element" relates to any nucleic acid introduced into the cell, which is not a component of the cells "original" or "natural" genome. Exogenous nucleic acids may be integrated or non-integrated or relate to stably transfected nucleic acids. The exogenous nucleic acid preferably encodes AAT, thereby leading to the expression or exogenous AAT protein.

### Exogenous protein:

As used herein, "exogenous protein" refers to protein expressed from a non-naturally occurring genetic element of a cell. For example, the coding nucleic acid encoding the exogenous protein is not present in the naturally occurring cell. For example, exogenous protein is expressed through exogenous DNA which integrates into the genome of expressing cell. The term exogenous AAT therefore relates to AAT protein not expressed from the existing (natural; endogenous) AAT gene(s), but rather expressed from an exogenous AAT nucleic acid or recombinantly produced and isolated for later use. In some embodiments, the AAT protein is considered exogenous when expressed from the naturally occurring coding sequence, but under control of a modified/transgenic promoter or other gene regulatory element.

As used herein "recombinant protein" is a protein coded by a recombinant gene and generated by the specific recombinant expression system. Recombinant proteins are important tools for studying biological processes and for industry manufacture of protein agents. Selection of an appropriate expression system is dependent on the characteristics and intended application of the recombinant protein and is essential to produce sufficient quantities of the protein. The commonly used expression systems are Escherichia coli, pichia pastoris, baculovirus, insect cell, and mammalian cells.

### Extra-cellular vesicles:

As used herein, "extra-cellular vesicles" (EVs) are lipid bilayer-delimited particles. They are naturally released from a cell and, unlike a cell, cannot replicate and range in diameter from near the size of the smallest physically possible unilamellar liposome (typically around 20-30 nanometers) to as large as 10 microns or more, although the majority of extra-cellular vesicles are smaller than 200 nm. They carry a cargo of proteins, nucleic acids, lipids, metabolites, and even organelles from the parent cell. Most cells that have been studied to date are thought to release extra-cellular vesicles, including some bacterial, fungal, and plant cells that are surrounded by cell walls. There are a wide variety of extra-cellular vesicles subtypes have been proposed, defined variously by size, biogenesis pathway, cargo, cellular source, and function, leading to a historically heterogenous nomenclature including terms like exosomes and ectosomes.

As used herein, "microvesicle" also called ectosomes and microparticles, is a type of extracellular vesicle that is released from the cell membrane. In multicellular organisms, microvesicles and other EVs are found both in tissues (in the interstitial space between cells) and in many types of body fluids. Delimited by a phospholipid bilayer, microvesicles can be as small as the smallest EVs (30 nm in diameter) or as large as 1000 nm. They are considered to be larger, on average, than intracellularly generated EVs known as exosomes. Microvesicles play a role in intercellular communication and can transport molecules such as mRNA, miRNA, and proteins between cells.

As used herein, "exosomes" are extracellular vesicles secreted by most eukaryotic cells and participate in intercellular communication. The components of exosomes, including proteins, DNA, mRNA, microRNA, long noncoding RNA, circular RNA, etc. They are a subset of extra-cellular vesicles surrounded by a lipid bilayer membrane and secreted by most eukaryotic cells and with a size of 1-500nm, preferably between about 10-200 nm, more preferably between 20-150nm nanometers in diameter.

As used herein, references to "extra-cellular vesicles" may be replaced by "exosomes" and vice versa.

As used herein, "concentration" as used herein is the abundance of a constituent divided by the total volume of a mixture. A concentration can be any kind of chemical mixture, but most frequently solutes and solvents in solutions. The molar (amount) concentration has variants such as normal concentration and osmotic concentration. The types of the concentration include mass concentration, molar concentration, number concentration, and volume concentration.

The concentration of extracellular vesicles or microvesicles or exosomes are determined by method from prior art, e.g. lysing the exosome followed by standard protein quantification, electron microscopy to determine particle size and hence to distinguish between exosomes and other vesicles, using for example Nanosight i.e. an optical microscopy adapted to quantify small particles, or for example ExoELISA i.e. an ELISA kit specific to quantify exosome particles.

Extra-cellular vesicles (EVs) have recently attracted substantial attention due to the potential diagnostic and therapeutic relevance. A variety of techniques have been used to isolate and analyze EVs. Size-exclusion chromatography (SEC), which separates subjects by size, has been widely applied in purification and analysis. Various other methods have been used for vesicle isolation, including ultracentrifugation, size exclusion (filtration or chromatography), immunoaffinity isolation, precipitation (ExoQuick or "salting-out") or the combinations of the above techniques. Techniques such as HPLC and/or ultrafiltration can also be employed to produce extra-cellular vesicles, such as exosomes.

As used herein, "high-performance liquid chromatography (HPLC)" formerly referred to as highpressure liquid chromatography, is a technique in analytical chemistry used to separate, identify, and quantify each component in a mixture. It comprises pumps to pass a pressurized liquid solvent containing the sample mixture through a column which is filled with a solid adsorbent material. Each component in the sample interacts slightly differently with the adsorbent material, generating different flow rates for the different components and resulting to the separation of the components as they flow out of the column. HPLC, for example size exclusion HPLC, can be employed to characterize and isolate extra-cellular vesicles, such as exosomes.

It has been shown previously that exosomes secreted from adipose derived mesenchymal stem cells can be obtained using with three common techniques: a stepwise ultracentrifugation at 100.000 g, an ultrafiltration with 3 kDa exclusion membranes and a charge-based precipitation method. Exosomes generated by mesenchymal stem cells can therefore be obtained by ultrafiltration and ultracentrifugation in a good quality for in vitro or in vivo experiments. For later therapeutic usage ultrafiltration is preferred due to a higher concentration without aggregation of extracellular vesicles in comparison to exosomes obtained by ultracentrifugation.

As used herein, "ultrafiltration" is a variety of membrane filtration in which forces like pressure or concentration gradients lead to a separation through a semipermeable membrane. Suspended solids and solutes of high molecular weight are retained in the retentate, while water and low molecular weight solutes pass through the membrane in the permeate. This separation process is used in industry and research for purifying and concentrating macromolecular solutions, especially protein solutions, extracellular vesicles, microvesicles and exosomes.

### Alpha-1 antitrypsin (AAT):

Alpha-1 antitrypsin (also known as A1AT, AAT, PI, SERPINA1) is a ∼52kDa glycoprotein that is one of the most abundant endogenous serine protease inhibitors (SERPIN superfamily). AAT is considered to be an acute phase protein, whereby AAT concentration can increase many fold upon acute inflammation.

The AAT encoding region is preferably any nucleic acid that encodes a naturally occurring or synthetic AAT protein sequence that exhibits AAT function, with reduced, the same, similar or increased activity compared to human AAT, or is functionally analogous to human AAT. The amino acid sequence of AAT is available under accession number 1313184B from the NCBI database. Corresponding nucleic acid sequences that encode AAT may be provided by one skilled in the art of molecular biology or genetics. The use of sequence variants of AAT that exhibit functional analogy or similarity to the unmodified human form of AAT is encompassed by the present invention.

The SERPINA1 coding sequence (CDS) as published at http://www.ncbi.nlm.nih.gov/nuccore/NM_000295.4 is one preferred embodiment and comprises bases 262 to 1518 of the full sequence (SEQ ID NO 1):

ATGCCGTCTTCTGTCTCGTGGGGCATCCTCCTGCTGGCAGGCCTGTGCTGCCTGGTCCCTG
TCTCCCTGGCTGAGGATCCCCAGGGAGATGCTGCCCAGAAGACAGATACATCCCACCATGA
TCAGGATCACCCAACCTTCAACAAGATCACCCCCAACCTGGCTGAGTTCGCCTTCAGCCTAT
ACCGCCAGCTGGCACACCAGTCCAACAGCACCAATATCTTCTTCTCCCCAGTGAGCATCGCT
ACAGCCTTTGCAATGCTCTCCCTGGGGACCAAGGCTGACACTCACGATGAAATCCTGGAGG
GCCTGAATTTCAACCTCACGGAGATTCCGGAGGCTCAGATCCATGAAGGCTTCCAGGAACT
CCTCCGTACCCTCAACCAGCCAGACAGCCAGCTCCAGCTGACCACCGGCAATGGCCTGTTC
CTCAGCGAGGGCCTGAAGCTAGTGGATAAGTTTTTGGAGGATGTTAAAAAGTTGTACCACTC
AGAAGCCTTCACTGTCAACTTCGGGGACACCGAAGAGGCCAAGAAACAGATCAACGATTAC
GTGGAGAAGGGTACTCAAGGGAAAATTGTGGATTTGGTCAAGGAGCTTGACAGAGACACAG
TTTTTGCTCTGGTGAATTACATCTTCTTTAAAGGCAAATGGGAGAGACCCTTTGAAGTCAAGG
ACACCGAGGAAGAGGACTTCCACGTGGACCAGGTGACCACCGTGAAGGTGCCTATGATGAA
GCGTTTAGGCATGTTTAACATCCAGCACTGTAAGAAGCTGTCCAGCTGGGTGCTGCTGATGA
AATACCTGGGCAATGCCACCGCCATCTTCTTCCTGCCTGATGAGGGGAAACTACAGCACCT
GGAAAATGAACTCACCCACGATATCATCACCAAGTTCCTGGAAAATGAAGACAGAAGGTCTG
CCAGCTTACATTTACCCAAACTGTCCATTACTGGAACCTATGATCTGAAGAGCGTCCTGGGT
CAACTGGGCATCACTAAGGTCTTCAGCAATGGGGCTGACCTCTCCGGGGTCACAGAGGAGG
CACCCCTGAAGCTCTCCAAGGCCGTGCATAAGGCTGTGCTGACCATCGACGAGAAAGGGAC
TGAAGCTGCTGGGGCCATGTTTTTAGAGGCCATACCCATGTCTATCCCCCCCGAGGTCAAG
TTCAACAAACCCTTTGTCTTCTTAATGATTGAACAAAATACCAAGTCTCCCCTCTTCATGGGA
AAAGTGGTGAATCCCACCCAAAAATAA

In some embodiments of the invention the CDS is codon optimized to increase protein production. The coding sequence after codon optimization preferably reads as follows (SEQ ID NO 2):

ATGCCCAGCAGCGTGTCCTGGGGAATTCTGCTGCTGGCCGGCCTGTGTTGTCTGGTGCCTG
TGTCTCTGGCCGAGGACCCTCAGGGGGATGCCGCCCAGAAAACCGATACCAGCCACCACG
ACCAGGACCACCCCACCTTCAACAAGATCACCCCCAACCTGGCCGAGTTCGCCTTCAGCCT
GTACAGACAGCTGGCCCACCAGAGCAACAGCACCAACATCTTTTTCAGCCCCGTGTCTATC
GCCACCGCCTTCGCCATGCTGAGCCTGGGCACAAAGGCCGACACCCACGACGAGATCCTG
GAAGGCCTGAACTTCAACCTGACCGAGATCCCCGAGGCCCAGATCCACGAGGGCTTCCAG
GAACTGCTGCGGACCCTGAACCAGCCCGATAGCCAGCTGCAGCTGACAACCGGCAACGGC
CTGTTTCTGAGCGAGGGACTGAAGCTGGTGGACAAGTTTCTGGAAGATGTGAAGAAGCTGT
ATCACAGCGAGGCCTTCACCGTGAACTTCGGCGACACCGAGGAAGCCAAGAAGCAGATCAA
CGACTACGTGGAAAAGGGCACCCAGGGCAAGATCGTGGACCTCGTGAAAGAGCTGGACCG
GGACACCGTGTTCGCCCTCGTGAACTACATCTTCTTCAAGGGCAAGTGGGAGCGGCCCTTC
GAAGTGAAGGACACAGAGGAAGAGGACTTTCACGTGGACCAAGTGACCACCGTGAAGGTG
CCCATGATGAAGAGACTGGGCATGTTCAACATCCAGCACTGCAAGAAACTGAGCAGCTGGG
TGCTGCTGATGAAGTACCTGGGCAACGCTACCGCCATATTCTTTCTGCCCGACGAGGGCAA

GCTGCAGCACCTGGAAAACGAGCTGACCCACGACATCATCACCAAATTTCTGGAAAATGAG
GACCGGCGGAGCGCCAGCCTGCATCTGCCTAAGCTGTCTATCACCGGCACCTACGACCTGA
AGTCCGTGCTGGGACAGCTGGGCATCACCAAGGTGTTCAGCAACGGCGCCGATCTGAGCG
GCGTGACAGAAGAGGCCCCTCTGAAGCTGTCCAAGGCCGTGCACAAAGCCGTGCTGACCA
TCGACGAGAAGGGCACCGAAGCCGCTGGCGCCATGTTTCTGGAAGCCATCCCCATGAGCAT
CCCCCCTGAAGTGAAGTTCAACAAGCCCTTCGTGTTCCTGATGATCGAGCAGAACACCAAGA
GCCCCCTGTTCATGGGCAAGGTCGTGAACCCCACCCAGAAA

The nucleotide sequence according to SEQ ID NO 1 and/or 2 encodes a human AAT protein of the amino acid sequence according to SEQ ID NO 3, which is preferred in the present invention:

The invention therefore encompasses use of a genetically modified MSC expressing transgenic AAT, as described herein comprising a nucleic acid molecule selected from the group consisting of:
a) a nucleic acid molecule comprising a nucleotide sequence that encodes AAT protein, such as according to SEQ ID NO 3, preferably by a nucleotide sequence according to SEQ ID NO 1 or 2,
b) a nucleic acid molecule which is complementary to a nucleotide sequence in accordance with a);
c) a nucleic acid molecule comprising a nucleotide sequence having sufficient sequence identity to be functionally analogous/equivalent to a nucleotide sequence according to a) or b), comprising preferably a sequence identity to a nucleotide sequence according to a) or b) of at least 70%, 80%, preferably 90%, more preferably 95%;
d) a nucleic acid molecule which, as a consequence of the genetic code, is degenerated into a nucleotide sequence according to a) through c); and/or
e) a nucleic acid molecule according to a nucleotide sequence of a) through d) which is modified by deletions, additions, substitutions, translocations, inversions and/or insertions and is functionally analogous/equivalent to a nucleotide sequence according to a) through d).

The invention therefore encompasses an AAT protein according to SEQ ID NO 3 or variants thereof, or the use of a genetically modified MSC as described herein comprising a nucleotide sequence encoding an amino acid sequence, according to SEQ ID NO 3.

The invention encompasses further sequence variants of SEQ ID NO 3, in particular those of at least 70% sequence identity to SEQ ID NO 3, preferably at least 80%, 85%, 90%, or at least 95% sequence identity to SEQ ID NO 3. Such sequence variants are preferably functionally analogous or equivalent to the human AA disclosed herein. Variations in the length of the protein are also encompassed by the invention, in cases where the functional equivalence of human AAT of SEQ ID NO 3 is maintained. Truncations or extensions in the length of the protein of, for example, up 50 amino acids, 40, 30, 20, or 10 amino acids may maintain AAT activity and are therefore encompassed in the present invention.

Functionally analogous sequences refer to the ability to encode a functional AAT gene product and to enable the same or similar functional effect as human AAT. AAT function may be determined by its ability to inhibit a variety of proteases in vitro, such as trypsin, or by its ability to inhibit neutrophil elastase (as described below). Appropriate assays for determining protease activity or for determining neutrophil elastase activity are known to a skilled person.

Protein modifications to the AAT protein, which may occur through substitutions in amino acid sequence, and nucleic acid sequences encoding such molecules, are also included within the scope of the invention. Substitutions as defined herein are modifications made to the amino acid sequence of the protein, whereby one or more amino acids are replaced with the same number of (different) amino acids, producing a protein which contains a different amino acid sequence than the primary protein. In some embodiments this amendment will not significantly alter the function of the protein. Like additions, substitutions may be natural or artificial. It is well known in the art that amino acid substitutions may be made without significantly altering the protein's function. This is particularly true when the modification relates to a "conservative" amino acid substitution, which is the substitution of one amino acid for another of similar properties. Such "conserved" amino acids can be natural or synthetic amino acids which because of size, charge, polarity and conformation can be substituted without significantly affecting the structure and function of the protein. Frequently, many amino acids may be substituted by conservative amino acids without deleteriously affecting the protein's function. In general, the non-polar amino acids Gly, Ala, Val, Ile and Leu; the non-polar aromatic amino acids Phe, Trp and Tyr; the neutral polar amino acids Ser, Thr, Cys, Gin, Asn and Met; the positively charged amino acids Lys, Arg and His; the negatively charged amino acids Asp and Glu, represent groups of conservative amino acids. This list is not exhaustive. For example, it is well known that Ala, Gly, Ser and sometimes Cys can substitute for each other even though they belong to different groups.

In other embodiments, the Alpha-1 antitrypsin (AAT) protein may be replaced by other serine protease inhibitors. Any feature with reference to AAT as disclosed herein may therefore also relate to other serine protease inhibitors. In one embodiment, the serine protease inhibitor is a trypsin-like serine protease inhibitor.

A trypsin-like serine protease inhibitor inhibits a serine protease with trypsin-like activity. Serine serves as the nucleophilic amino acid at the enzyme's active site of the serine protease enzyme. Numerous trypsin-like serine proteases have been under active pursuit as therapeutic targets for inhibition. Viral entry via endocytosis is dependent on target cell proteases, such as serine proteases.

Viral infections are target cell serine protease dependent if the virus requires a target cell serine protease for viral entry and growth.

The target cell serine protease activates the virus Spike protein required for fusion of the viral and target cell membranes and the release of the viral genome into the cytosol of the target cell. Targeting the host cell serine protease can prevent viral growth of an endocytosis dependent virus, whereby inhibiting the target cell serine protease is an effective method for treating a subject infected with the endocytosis dependent virus. The serine protease inhibitor, preferably a trypsin-like serine protease inhibitor, inhibits viral infection and virus growth. As non-limiting examples, trypsin-like serine protease inhibitors include camostat, aprotinin, benzamidine, gabexate, leupeptin, nafamostat, pepstatin A, ribavirin, sepimostat, ulinastatin, and patamostat.

### Medical Indications:

In one aspect of the present invention there is provided a pharmaceutical composition or combination as herein described for use in the treatment and/or prevention of a viral infection in a subject and/or a medical condition associated with a viral infection. Preferred viral infections to be treated or associated diseases are those described herein.

As used herein, "viral infection" occurs when an organism's body is invaded by pathogenic viruses. The virus type is preferably selected from the group consisting of adenovirus, coxsackievirus, Epstein-barr virus, Hepatitis A virus, hepatitis B virus, hepatitis C virus, Herpes simplex virus type 1, herpes simplex virus type 2, cytomegalovirus, human herpesvirus type 8, HIV, influenza virus, measles virus, mumps virus, human papillomavirus, poliovirus, rabies virus, respiratory syncytial virus, rubella virus, varicella-zoster virus, SARS-CoV-2 virus. In one embodiment, the family of the virus is preferably selected from the group of adenoviridae, picornaviridae, Herpesviridae, picornaviridae, hepadnaviridae, Flaviviridae, Herpesviridae, retroviridae, Orthomyxoviridae, paramyxoviridae, papillomavirus, picornaviridae, Rhaboviridae, Togaviridae, Herpesviridae and coronaviridae.

In one aspect of the present invention there is provided a pharmaceutical composition or combination according to the invention as described herein for use in the treatment of a medical condition associated with a SARS Coronavirus, wherein the medical condition associated with a SARS Coronavirus is preferably COVID-19 or a SARS Coronavirus-associated respiratory disease.

As used herein, the "patient" or "subject" may be a vertebrate. In the context of the present invention, the term "subject" includes both humans and animals, particularly mammals, and other organisms.

In the present invention "treatment" or "therapy" generally means to obtain a desired pharmacological effect and/or physiological effect. The effect may be prophylactic in view of completely or partially preventing a disease and/or a symptom, for example by reducing the risk of a subject having a disease or symptom or may be therapeutic in view of partially or completely curing a disease and/or adverse effect of the disease.

In the present invention, "therapy" includes arbitrary treatments of diseases or conditions in mammals, in particular, humans, for example, the following treatments (a) to (c): (a) Prevention of onset of a disease, condition or symptom in a patient; (b) Inhibition of a symptom of a condition, that is, prevention of progression of the symptom; (c) Amelioration of a symptom of a condition, that is, induction of regression of the disease or symptom.

In one embodiment, the treatment described herein relates to either reducing or inhibiting Coronavirus infection or symptoms thereof via preventing viral entry into target cells. The prophylactic therapy as described herein is intended to encompass prevention or reduction of risk of Coronavirus infection, due to a reduced likelihood of Coronavirus infection of cells via interaction with the ACE2 protein after treatment with the agents described herein.

As used herein, a "patient with symptoms of an infectious disease" is a subject who presents with one or more of, without limitation, fever, diarrhea, fatigue, muscle aches, coughing, if have been bitten by an animal, having trouble breathing, severe headache with fever, rash or swelling, unexplained or prolonged fever or vision problems. Other symptoms may be fever and chills, very low body temperature, decreased output of urine (oliguria), rapid pulse, rapid breathing, nausea and vomiting. In preferred embodiments the symptoms of an infectious disease are fever, diarrhea, fatigue, muscle aches, rapid pulse, rapid breathing, nausea and vomiting and/or coughing.

As used herein, a patient with "symptoms of a viral infection of the respiratory tract" is a subject who presents with one or more of, without limitation, cold-like symptoms or flu-like illnesses, such as fever, cough, runny nose, sneezing, sore throat, having trouble breathing, headache, muscle aches, fatigue, rapid pulse, rapid breathing, nausea and vomiting, lack of taste and/or smell and/or malaise (feeling unwell).

In some embodiments, symptoms of infection with a SARS-virus are fever, sore throat, cough, myalgia or fatigue, and in some embodiments, additionally, sputum production, headache, hemoptysis and/or diarrhea. In some embodiments, symptoms of an infection with a SARS-coronavirus, for example SARS-CoV-2, are fever, sore throat, cough, lack of taste and/or smell, shortness of breath and/or fatigue.

As used herein, the term "a patient that is at risk of developing a severe acute respiratory syndrome (SARS)" relates to a subject, preferably distinct from any given person in the general population, who has an increased (e.g. above-average) risk of developing SARS. In some embodiments, the patient has symptoms of SARS or symptoms of a SARS Coronavirus infection. In some embodiments, the patient has no symptoms of SARS or symptoms of a SARS Coronavirus infection. In some embodiments, the subject has been in contact with people with SARS Coronavirus infections or symptoms. In some embodiments, the person at risk of developing SARS has been tested for the presence of a SARS Coronavirus infection. In some embodiments, the person at risk of developing SARS has tested positive for the presence of a SARS Coronavirus infection, preferably a coronavirus infection.

In embodiments, the patient at risk of developing SARS is an asymptomatic patient that shows no specific symptoms of SARS (yet). An asymptomatic patient may be at risk of developing SARS because the patient has been in contact with a person infected with a SARS Coronavirus. For example, the asymptomatic patient may have been identified as being at risk of developing SARS by a software application (app) that is installed on his smart phone or corresponding (portable) device and that indicates physical proximity or short physical distance to an infected patient that uses a corresponding app on its respective mobile device/smart phone. Other methods of determining contact/physical proximity to an infected person are known to the skilled person and equally apply to the method of the invention.

In some embodiments, the patient that has or is at risk of developing a severe acute respiratory syndrome (SARS) has a coronavirus infection.

Coronaviruses are a group of related viruses that cause diseases in mammals and birds. The scientific name for coronavirus is Orthocoronavirinae or Coronavirinae. Coronavirus belongs to the family of Coronaviridae. The family is divided into Coronavirinae and Torovirinae sub-families, which are further divided into six genera: Alphacoronavirus, Betacoronavirus, Gammacoronavirus, Deltacoronavirus, Torovirus, and Bafinivirus. While viruses in the genera Alphacoronaviruses and Betacoronaviruses infect mostly mammals, the Gammacoronavirus infect avian species and members of the Deltacoronavirus genus have been found in both mammalian and avian hosts.

In humans, coronaviruses cause respiratory tract infections that can be mild, such as some cases of the common cold, and others that can be lethal, such as SARS, MERS, and COVID-19. Coronaviruses are enveloped viruses with a positive-sense single-stranded RNA genome and a nucleocapsid of helical symmetry. The genome size of coronaviruses ranges from approximately 27 to 34 kilobases, the largest among known RNA viruses.

Various species of human coronaviruses are known, such as, without limitation, Human coronavirus OC43 (HCoV-OC43), of the genus β-CoV, Human coronavirus HKU1 (HCoV-HKU1), of the genus β-CoV, Human coronavirus 229E (HCoV-229E), α-CoV, Human coronavirus NL63 (HCoV-NL63), α-CoV, Middle East respiratory syndrome-related coronavirus (MERS-CoV), Severe acute respiratory syndrome coronavirus (SARS-CoV), Severe acute respiratory syndrome coronavirus 2 (SARS-CoV-2).

Coronaviruses vary significantly in risk factor. Some can kill more than 30% of those infected (such as MERS-CoV), and some are relatively harmless, such as the common cold. Coronaviruses cause colds with major symptoms, such as fever, and a sore throat, e.g. from swollen adenoids, occurring primarily in the winter and early spring seasons. Coronaviruses can cause pneumonia (either direct viral pneumonia or secondary bacterial pneumonia) and bronchitis (either direct viral bronchitis or secondary bacterial bronchitis). Coronaviruses can also cause SARS.

Advances in nucleic acid sequencing technology (commonly termed Next-Generation Sequencing, NGS) are providing large sets of sequence data obtained from a variety of biological samples and allowing the characterization of both known and novel virus strains. Established methods are therefore available for determining a Coronavirus infection.

### Compositions and administration:

According to the invention, administration of the composition may preferably occur topically and/or locally to a mucosal surface of the respiratory tract and/or oral cavity of a subject. "Topical administration" is the application of a drug to a certain area of the skin or mucosal surface or other tissue locally so that the agent will take effect on the surface and/or on the underlying tissue. "Local administration" is administration to a specific and limited area, preferably by topical administration.

As used herein, the "respiratory tract" is commonly considered to comprise the nose, pharynx, larynx, trachea, and the lung with its different compartments. The respiratory tract is involved with the process of respiration in mammals, and is a part of the respiratory system, and is lined with respiratory mucosa or respiratory epithelium.

As used herein, a "mucosal surface" is characterised by the presence of an overlying mucosal fluid, e.g. saliva, tears, nasal, gastric, cervical and bronchial mucus, the functions of which include to supply and deliver an array of immunoregulatory and pro-healing species including growth factors, antimicrobial proteins and immunoglobulins.

As used herein, a "pharmaceutical excipient" is essentially anything other than the active pharmaceutical ingredient in a composition.

In the formulation of pharmaceutical compositions, excipients are generally added along with the active pharmaceutical ingredients in order to 1) protect, support or enhance the stability of the formulation; 2) bulk up the formulation in case of potent drug for assisting in the formulation of an accurate dosage form; 3) improve patient acceptance; 4) help improve bioavailability of active drugs; 5) enhance overall safety and effectiveness of the formulation during its storage and use.

Excipients used in the formulation of pharmaceutical compositions are sub-divided into various functional classifications, depending on the role they intend to play in the resultant formulation. Some excipients can have different functional roles in different formulation types and in addition, individual excipients can have different grades, types, and sources depending on those different functional roles. The possible types of excipients commonly used in the formulation of pharmaceutical suspensions include solvents/vehicle, co-solvent, buffering agents, preservatives, antioxidants, wetting agents/surfactants, anti-foaming agents, flocculation modifiers, suspending agents/viscosity-modifiers, flavouring agents, sweetening agents, colorants, humectants, and chelating agents.

As used herein, "cryopreservation" is a process where organelles, cells, tissues, extracellular matrix, organs, or any other biological constructs susceptible to damage caused by unregulated chemical kinetics are preserved by cooling to very low temperatures by freezing (typically -20 to - 80 °C using solid carbon dioxide or in some cases to -196 °C using liquid nitrogen). At low enough temperatures, any enzymatic or chemical activity which might cause damage to the biological material in question is effectively stopped. Cryopreservation methods seek to reach low temperatures without causing additional damage caused by the formation of ice crystals during freezing.

As used herein, an "aerosol" is a suspension of liquid and solid particles produced for example by an aerosol generator, such as a small-volume nebulizer (SVN), a pressurized metered-dose inhaler (pMDI), or a dry-powder inhaler (DPI). Aerosol deposition is a process of aerosol particles depositing on absorbing surfaces.

Aerosol devices in clinical use produce heterodisperse (also termed polydisperse) particle sizes, meaning that there is a mix of sizes in the aerosol. Specific sizes and/or size ranges are presented in the Summary of the invention above. Monodisperse aerosols, which consist of a single particle size, are rare. Polydisperse aerosols can be defined by the mass median diameter (MMD). This measure determines the particle size (in µm) above and below which 50% of the mass of the particles is contained. This is the particle size that evenly divides the mass, or amount of the drug in the particle size distribution. This is usually given as the mass median aerodynamic diameter, or MMAD, due to the way sizes are measured. The higher the MMAD, the more particle sizes are of larger diameters.

Dry powder formulations can offer advantages, including greater stability than liquid formulations and potential that preservatives may not be required. Powders tend to stick to the moist surface of the nasal mucosa before being dissolved and cleared. The use of bio-adhesive excipients or agents that slow ciliary action may decrease clearance rates and improve absorption. A number of factors like moisture sensitivity, solubility, particle size, particle shape, and flow characteristics will impact deposition and absorption.

### Examples of dry powder nasal sprays:

SBNL Pharma (www.snbl.com) recently reported data on a Phase 1 study with a zolmitriptan powder cyclodextrin formulation (µcοTM) System) for enhanced absorption, described previously in an in vitro study. The zolmitriptan absorption was rapid, and the relative bioavailability was higher than the marketed tablet and nasal spray. The company has a capsule-based, single-dose powder device (Fit-lizer), suitable for administration of a powder formulation according to the present invention. When inserted into a chamber, the top and bottom of the capsule is cut off by sharp blades. A plastic chamber is compressed by hand, compressed air passes through a one-way valve and the capsule during actuation, and the powder is emitted.

Bespak (www.bespak.com), the principle for Unidose-DPTM, is similar to the Fit-lizer device. An air-filled compartment is compressed until a pin ruptures a membrane to release the pressure to emit the plume of powder. Delivery of powder formulations of a model antibody (human IgG) has been tested in a nasal cast model based on human MRI images. Approximately 95 % of the dose was delivered to the nasal cavity, but the majority of it was deposited no further than the nasal vestibule with only about 30 % deposited into deeper compartments of the nasal cavity.

### Examples of dry powder inhalers:

Astra Zeneca markets budesonide powder delivered with the Turbuhaler multi-dose inhaler device modified for nasal inhalation (Rhinocort Turbuhaler^{®}; www.az.com). It is marketed for allergic rhinitis and nasal polyps in some markets as an alternative to the liquid spray.

The Aptar group (www.aptar.com) offers a simple blister-based powder inhaler. The blister is pierced before use and the device nosepiece placed into one nostril. The subject closes the other nostril with the finger and inhales the powder into the nose. A powder formulation of apomorphine for Parkinson's using this blister-based powder inhaler (BiDoseTM/ProhalerTM) from Pfeiffer/ Aptar was in clinical development by Britannia, a UK company recently acquired by Stada Pharmaceutical (www.stada.de). Such administration devices are contemplated for administering the composition as described herein.

### Examples of inhalers with liquid formulations:

Inhalation solution and suspension drug products are typically aqueous-based formulations that contain therapeutically active ingredients and can also contain additional excipients. Aqueous-based oral inhalation solutions and suspension must be sterile. Inhalation solutions and suspensions are intended for delivery to the lungs by oral inhalation for local and/or systemic effects and are to be used with a specified nebulizer. Unit-dose presentation is recommended for these drug products to prevent microbial contamination during use. The container closure system for these drug products consists of the container and closure and can include protective packaging such as foil overwrap.

For example, amikacin liposome inhalation suspension (ALIS; Arikayce^{®}) is a liposomal formulation of the aminoglycoside antibacterial drug amikacin. The ALIS formulation, administered via inhalation following nebulization, is designed to facilitate targeted and localized drug delivery to the lungs while minimizing systemic exposure. Such administration devices are contemplated for administering the composition as described herein.

### Examples of nasal sprays with liquid formulation:

Nasal spray dispensers are typically non-pressurized dispensers that deliver a spray containing a metered dose of the active ingredient. The dose can be metered by the spray pump or could have been pre-metered during manufacture. A nasal spray unit can be designed for unit dosing or can discharge up to several hundred metered sprays of formulation containing the drug substance. Nasal sprays are applied to the nasal cavity for local and/or systemic effects.

The liquid nasal formulations are mainly aqueous solutions, but suspensions and emulsions can also be delivered. Liquid formulations are considered convenient particularly for topical indications where humidification counteracts the dryness and crusting often accompanying chronic nasal diseases.

For example, the dispensers available for example from Nemera (La Verpilliere, France) or Aptar Pharma (Illinois, USA) are preferred. For example, Nemera provides Advancia^{®} nasal spray dispensers, which are high-performing pumps with good dose consistency and prime retention, anti-clogging actuators, no metal contacting the formulation and hygienic anti-actuation snap-on overcaps. As a further example, Aptar Pharma's nasal pump technology removes the need for drug manufacturers to add preservatives to nasal spray formulations. The Advanced Preservative Free (APF) systems use a tip-seal and filter technology to prevent contamination of the formulation. A spring-loaded tip seal mechanism is employed with a filter membrane in the ventilation channel.

Metering and spray producing (e.g., orifice, nozzle, jet) pump mechanisms and components are used for reproducible delivery of drug formulation, and these can be constructed of many parts of different design that are precisely controlled in terms of dimensions and composition. Energy is required for dispersion of the formulation as a spray. This is typically accomplished by forcing the formulation through the nasal actuator and its orifice. The formulation and the container closure system (container, closure, pump, and any protective packaging) collectively constitute the drug product. The design of the container closure system affects the dosing performance of the drug product.

In some embodiments, the multi-use dispenser employs a membrane (preferably of silicone) that prevents bacteria or viruses from entering the reservoir or pump device. In some embodiments, the multi-use dispenser employs a metal-free fluid path, thereby preventing the oxidization of the formulation. In some embodiments, the multi-use dispenser employs a spring-loaded tip seal mechanism, thereby preventing microbes from entering the device between spray events.

In one embodiment, the dispenser is configured for multiple individual spray events of 5 to 1000 µL, preferably 5 to 500 µL, more preferably 10 to 300 µL, more preferably 20 to 200 µL.

In one embodiment, the dispenser comprises a total volume of composition of 0.1 to 500 mL, preferably 1 to 100 mL, more preferably 2 to 50 mL, such as for example about 5, 10 or 15 mL.

### Examples of throat sprays for liquid formulations:

Bona, ShenZhen China, is a supplier of medical packaging and complements. The firm offers a line of long-neck sprays that offer excellent dispensing and dosing, designed for throat sprays and other treatments that require throat targeting.

The arm of the dispenser swings 360 degrees for consumer ease of use. The sprayers are suited for medicated throat treatments as well as other treatments that local treatment, for example to protect the throat from viral infections. The dispensers are preferably prepared from pharma-grade PP and PE, and the dispensers can be paired with any bottle size. Currently, the sprayers come in a variety of popular sizes (18/410,18/415, 20/410, 24/410) and can be set to dispense from 220 microliters to 50 microliters.

### Pharmaceutical Combinations:

The present invention relates to a pharmaceutical composition, and in other embodiments, to a pharmaceutical combination. According to the present invention, a "pharmaceutical combination" preferably relates to the combined presence of extracellular vesicles from MSCs and AAT protein, or the use of the two components in combined administration. In one embodiment, the combination is suitable for combined administration.

In one embodiment, the pharmaceutical combination as described herein is characterized in that the extracellular vesicles from MSCs are in a pharmaceutical composition in admixture with a pharmaceutically acceptable carrier, and the AAT is in a separate pharmaceutical composition in admixture with a pharmaceutically acceptable carrier. The pharmaceutical combination of the present invention can therefore in some embodiments relate to the presence of two separate compositions or dosage forms in proximity to each other. The agents in combination are not required to be present in a single composition.

In one embodiment, the pharmaceutical combination as described herein is characterized in that the extracellular vesicles from MSCs and the AAT protein as described herein are present in a kit, in spatial proximity but in separate containers and/or compositions. The production of a kit lies within the abilities of a skilled person. In one embodiment, separate compositions comprising two separate agents may be packaged and marketed together as a combination. In other embodiments, the offering of the two agents in combination, such as in a single catalogue, but in separate packaging is understood as a combination.

In one embodiment, the pharmaceutical combination as described herein is characterized in that the extracellular vesicles from MSCs and the AAT protein as described herein are combined in a single pharmaceutical composition in admixture with a pharmaceutically acceptable carrier. Combination preparations or compositions are known to a skilled person, who is capable of assessing compatible carrier materials and formulation forms suitable for both agents in the combination.

### Combined administration:

According to the present invention, the term "combined administration", otherwise known as coadministration or joint treatment, encompasses in some embodiments the administration of separate formulations of the compounds described herein, whereby treatment may occur together, within minutes of each other, in the same hour, on the same day, in the same week or in the same month as one another. Alternating administration of two agents is considered as one embodiment of combined administration. Staggered administration is encompassed by the term combined administration, whereby one agent may be administered, followed by the later administration of a second agent, optionally followed by administration of the first agent, again, and so forth. Simultaneous administration of multiple agents is considered as one embodiment of combined administration. Simultaneous administration encompasses in some embodiments, for example the taking of multiple compositions comprising the multiple agents at the same time, e.g. orally by ingesting separate tablets simultaneously. A combination medicament, such as a single formulation comprising multiple agents disclosed herein, and optionally additional anti-cancer medicaments, may also be used in order to co-administer the various components in a single administration or dosage.

A combined therapy or combined administration of one agent may occur together or precede or follow treatment with the other agent to be combined, by intervals ranging from minutes to weeks. In embodiments where the second agent and the first agent are administered separately, one would generally ensure that a significant period of time did not expire between the time of each delivery, such that the first and second agents would still be able to exert an advantageously combined synergistic effect on a treatment site. In such instances, it is contemplated that one would contact the subject with both modalities within about 12-24 h of each other and, more preferably, within about 6-12 h of each other, with a delay time of only about 12 h being most preferred. In some situations, it may be desirable to extend the time period for treatment significantly, however, where several days (2, 3, 4, 5, 6 or 7) to several weeks (1, 2, 3, 4, 5, 6, 7 or 8) lapse between the respective administrations.

In the meaning of the invention, any form of administration of the multiple agents described herein is encompassed by combined administration, such that a beneficial additional therapeutic effect, preferably a synergistic effect, is achieved through the combined administration of the two agents.

### Synergy:

In some embodiments, the combination of extracellular vesicles from MSCs and AAT protein leads to a synergistic effect.

To determine or quantify the degree of synergy or antagonism obtained by any given combination, a number of models may be employed. Typically, synergy is considered an effect of a magnitude beyond the sum of two known effects. In some embodiments, the combination response is compared against the expected combination response, under the assumption of non-interaction calculated using a reference model (refer Tang J. et al. (Tang, Wennerberg, and Aittokallio 2015))

Commonly utilized reference models include the Highest single agent (HSA) model (Berenbaum 1989), the Loewe additivity model (LOEWE 1953), the Bliss independence model (BLISS 1939), and more recently, the Zero interaction potency (ZIP) model (Yadav et al. 2015). The assumptions being made in these reference models are different from each other, which may produce somewhat inconsistent conclusions about the degree of synergy. Nevertheless, according to the present invention, when any one of these models indicates synergy between the agents in the combination as described herein, it may be assumed synergy has been achieved. Preferably, 2, 3 or all 4 of these models will reveal synergy between any two agents of the combination described herein.

Without limitation, four reference models are preferred, which can produce reliable results: (i) HSA model, where the synergy score quantifies the excess over the highest single drug response; (ii) Loewe model, where the synergy score quantifies the excess over the expected response if the two drugs are the same compound; (iii) Bliss model, where the expected response is a multiplicative effect as if the two drugs act independently; and (iv) ZIP model, where the expected response corresponds to an additive effect as if the two drugs do not affect the potency of each other.

The most widely used combination reference, and preferred model for determining synergy, is "Loewe additivity", or the "Loewe model" (Loewe 1928; LOEWE 1953; Loewe and Muischnek 1926), or "dose additivity" which describes the trade-off in potency between two agents when both sides of a dose matrix contain the same compound. For example, if 50% inhibition is achieved separately by 1 uM of drug A or 1 uM of drug B, a combination of 0.5 uM of A and 0.5 uM of B should also inhibit by 50%. Synergy over this level is especially important when justifying the clinical use of proposed combination therapies, as it defines the point at which the combination can provide additional benefit over simply increasing the dose of either agent.

### FIGURES

The following figures are presented to describe particular embodiments of the invention, without being limiting in scope.
- **Fig. 1:**: AAT is expressed from transgenes encoding AAT from genetically modified MSCs.
- **Fig. 2:**: AAT expressed from genetically modified MSCs and from human plasma is equally potent in inhibiting neutrophil elastase.
- **Fig. 3:**: Schematic of in vivo experiment for the treatment of elastase-induced emphysema in a mouse model.
- **Fig. 4:**: In vivo efficacy of MSCs transduced to express AAT for the treatment of elastase-induced emphysema in a mouse model.
- **Fig. 5:**: Lung tissue repair and regeneration assessed by histological analysis.

### EXAMPLES

The following examples are presented to describe particular embodiments of the invention, without being limiting in scope.

### AAT expression from genetically modified MSCs:

Primary human MSCs derived from 4 healthy donors were transduced with an optimized retroviral construct (n = 36). Transduced cells were selected with puromycin and expanded according to established protocols. Protein secreted from genetically modified MSCs - including the form of secreted extra-cellular vesicles - was quantified in the cell culture supernatant by an ELISA specifically detecting human AAT. AAT-MSCs produce an average of ∼650ng AAT/1x105 cells/VC in 48h.

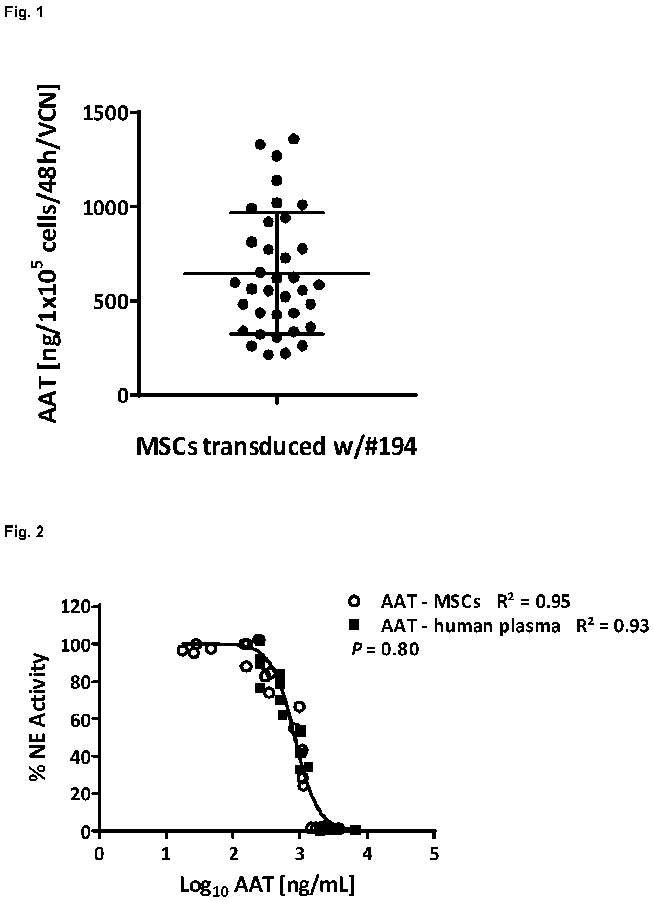

Results are shown in Fig. 1.

### AAT expressed from genetically modified MSCs vs AAT from human plasma:

Supernatants of transduced MSCs - including secreted extra-cellular vesicles - were incubated with neutrophil elastase before the addition of a fluorescent elastase substrate. In case of inhibition of neutrophil elastase by the supernatant, hydrolyzation of the substrate was reduced, resulting in a decreased RFU output. Natural full-length AAT from human serum was tested as a positive control; DMEM and supernatant from non-transduced MSCs served as negative controls. AAT derived from transduced MSCs and from human plasma is equally potent in inhibiting neutrophil elastase.

Results are shown in Fig. 2.

### MSCs expressing transgenic AAT for the treatment of elastase-induced emphysema:

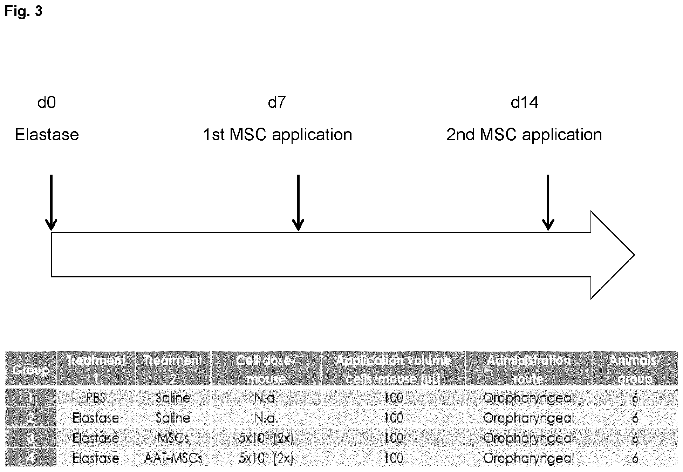

The in vivo efficacy of MSCs transduced to express AAT were assessed in the treatment of elastase-induced emphysema in a mouse model. The model outline is demonstrated in Figure 3.

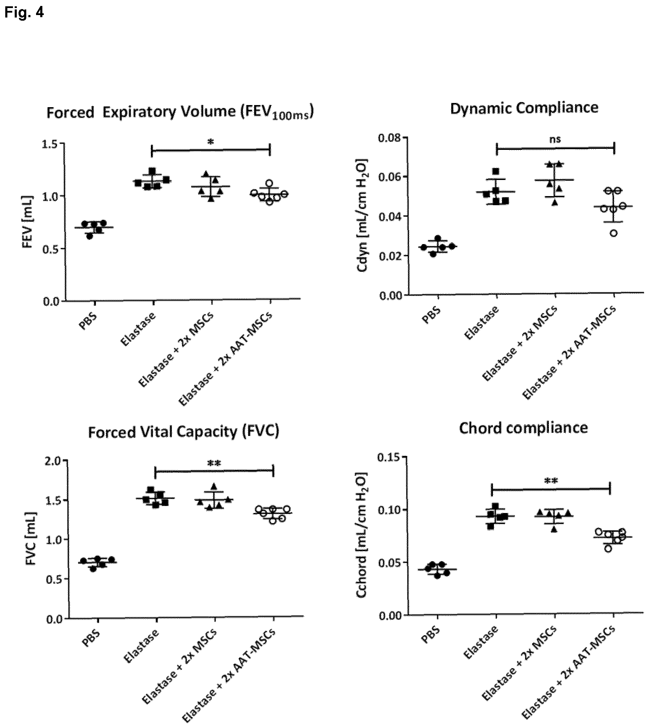

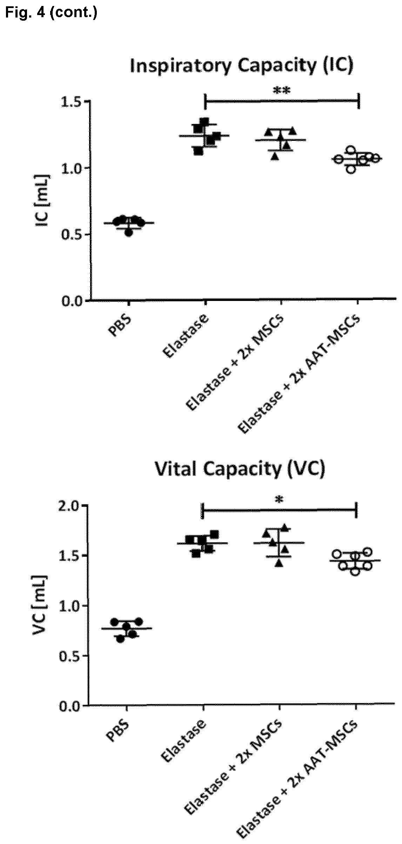

As can be seen in Figure 4, a statistically significant amelioration of 5 out of 6 lung function parameters was achieved by AAT-MSCs, not by native MSCs.

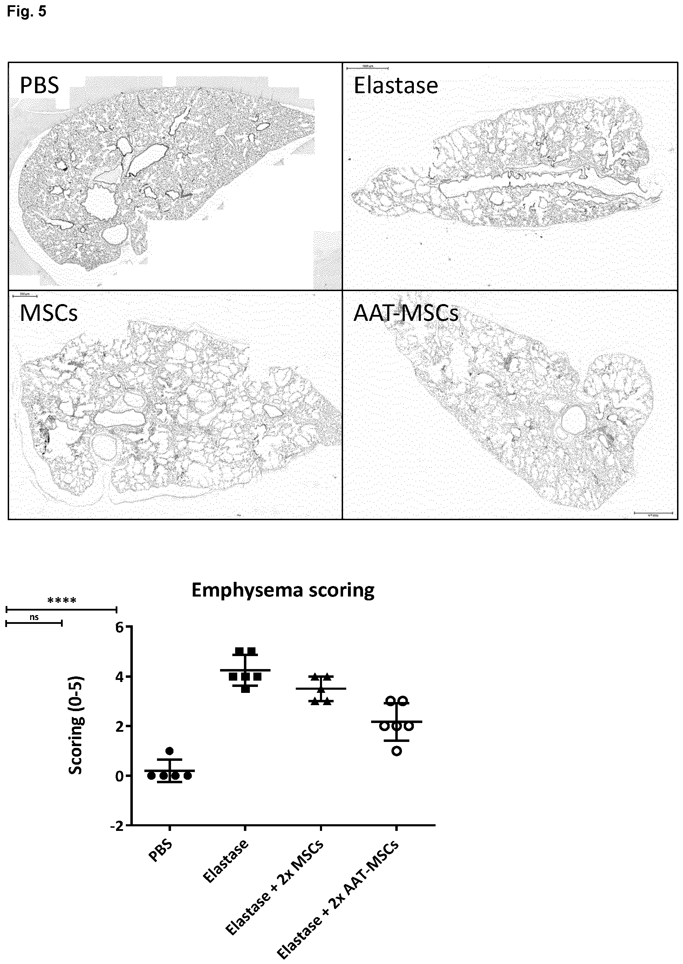

Lung tissue repair and regeneration was assessed by histological analysis after treatment with unmodified MSCs or AAT-MSCs. As shown in Figure 5, significant lung tissue repair/regeneration after 2-fold application of AAT-MSCs.

### Summary.

As shown above, AAT is effectively expressed in MSCs from an exogenous gene and shows similar activity to AAT isolated from human plasma. The treatment of the elastase-induced emphysema lung model as described herein represents support for AAT-expression and secretion from MSCs and the desirable anti-inflammatory properties achieved therein, in particular with respect to lung tissue.

Direct assessment of effects against viral infection when using isolated MSC extra-cellular vesicles combined with AAT, either enriched or expressed from MSCs, is ongoing.

## Claims

1. A pharmaceutical composition comprising extra-cellular vesicles isolated from mesenchymal stem cells (MSCs) and Alpha-1 antitrypsin (AAT) protein, for use as a medicament in the treatment and/or prevention of a viral infection in a human subject.

2. The pharmaceutical composition for use according to claim 1, wherein the AAT protein is expressed in the mesenchymal stem cells (MSCs) from which the extra-cellular vesicles are isolated, wherein the MSCs are genetically modified to express exogenous Alpha-1 antitrypsin (AAT).

3. The pharmaceutical composition for use according to any one of the preceding claims, wherein the composition is enriched with Alpha-1 antitrypsin (AAT) protein, preferably enriched with isolated recombinant AAT protein.

4. The pharmaceutical composition for use according to any one of the preceding claims, wherein said composition is administered topically and/or locally to a mucosal surface of the respiratory tract and/or oral cavity of the subject.

5. The pharmaceutical composition for use according to any one of the preceding claims, wherein the extra-cellular vesicles are exosomes and/or microvesicles.

6. The pharmaceutical composition for use according to any one of the preceding claims, wherein the extra-cellular vesicles are exosomes, which are obtainable by a method comprising the following steps:
a) Providing a cell culture supernatant comprising exosomes from mesenchymal stem cells (MSCs), optionally genetically modified to express exogenous Alpha-1 antitrypsin (AAT); and
b) Isolating the exosomes by removing the cells and/or cell debris, preferably by centrifugation, high-performance liquid chromatography (HPLC) and/or ultrafiltration.

7. The pharmaceutical composition for use according to any one of the preceding claims, wherein said MSCs are selected from human MSCs derived from induced pluripotent stem cells (iPSCs), umbilical cord blood, umbilical cord tissue, placenta, bone marrow, bone or adipose tissue and MSCs that have been cultured in platelet lysate.

8. The pharmaceutical composition for use according to any one of the preceding claims, wherein the extra-cellular vesicles have an average diameter of 1-500nm, preferably between about 10 to 200 nm, more preferably between 20 to 150nm.

9. The pharmaceutical composition for use according to any one of the preceding claims, wherein the concentration of the extra-cellular vesicles is 0.01-100 mg/ml, preferably 0.1-10 mg/ml, more preferably 1-5 mg/ml.

10. The pharmaceutical composition for use according to any one of the preceding claims, wherein the extra-cellular vesicles comprise AAT protein, preferably exogenous AAT expressed form the modified MSCs, and one or more biological factors selected from the group consisting of cytokines, such as IFN-γ, IL-8, IL-10 and TGF-β, HLA-G, cholesterol, ceramide, phosphoglycerides, saturated fatty-acyl chains, prostaglandins and nucleic acids, such as RNA, preferably miRNA, siRNA and shRNA.

11. The pharmaceutical composition for use according to any one of the preceding claims, wherein the composition is suitable for administration to the upper or lower respiratory tract, the lungs, nasal cavity, oral cavity and/or throat of a subject.

12. The pharmaceutical composition for use according to any one of the preceding claims, wherein said composition is in liquid or powder form and is suitable for spraying in the nasal and/or oral cavity of a subject and/or for inhalation.

13. The pharmaceutical composition for use according to any one of the preceding claims, wherein the subject is suspected of having a viral infection and/or has been in proximity and/or in contact with an individual with a viral infection.

14. The pharmaceutical composition for use according to any one of the preceding claims, wherein the virus is SARS-CoV-2.

15. A delivery system for delivering a pharmaceutical composition according to any one of the preceding claims to a subject, the system comprising:
a) a pharmaceutical composition according to any one of the preceding claims, with one or more pharmaceutical excipient(s), and
b) a dispenser, wherein the dispenser is suitable for spraying the composition in the nasal and/or oral cavity of a subject and/or for inhalation of the composition, and is preferably formulated for cryopreservation without significant functional loss of the isolated extra-cellular vesicles.
